# EUROPEAN PATENT APPLICATION

(11) **EP 4 371 558 A1**
(43) Date of publication of application: **22.05.2024**
(21) Application number: 22841360.5
(22) Date of filing: 12.07.2022
(51) Int. Cl.: A61K 9/22, A61K 31/41, A61K 31/216, A61K 9/44, A61K 9/36, A61K 47/36, A61K 47/38, A61P 9/04

(54) **SACUBITRIL VALSARTAN SODIUM SUSTAINED-RELEASE COMPOSITION, AND PREPARATION METHOD THEREFOR AND USE THEREOF**

(30) Priority: 12.07.2021 CN 202110784642
(71) Applicant: Shanghai Aurora Biotechnology Co., Ltd., Shanghai 201210 (CN)
(72) Inventor: GUO, Zhen, Shanghai 201210 (CN); XIE, Wenfeng, Shanghai 201210 (CN); XIAO, Xiaoling, Shanghai 201210 (CN); WANG, Tingting, Shanghai 201210 (CN); YING, Shuhuan, Shanghai 201210 (CN)
(74) Representative: Gille Hrabal Partnerschaftsgesellschaft mbB Patentanwälte
(86) International application number: PCT/CN2022/105144
(87) International publication number: WO 2023/284724

(57) **Abstract**

A sacubitril valsartan sodium sustained-release composition, and a preparation method therefor and the use thereof. The composition is a 24-hour sustained-release drug. Dissolution of sacubitril and valsartan simultaneously satisfies the following three features: A) dissolving no more than 40% of pharmaceutical active ingredients within 2 hours; B) dissolving 20%-75% of the pharmaceutical active ingredients within 8 hours; and C) dissolving not less than 65% of the pharmaceutical active ingredients within 24 hours. Compared with common gel sustained-release dosage forms, sacubitril and valsartan in the composition can achieve synchronous sustained release; moreover, the drug release behavior is not affected by factors such as the pH of a medium environment, gastrointestinal peristalsis, and foods, the in vivo and in vitro correlation is good, gentle release within 24 hours is achieved, and the therapeutic effect is good. Compared with common immediate-release formulations, the medication frequency is reduced, the patient compliance is improved, the medication compliance is improved for people having difficulty in swallowing such as the elderly and children, it is convenient to medicate, the toxic side effects are small, and the market prospects are good.

## Description

The present application claims the priority to Patent Application No. 202110784642.5, entitled "SACUBITRIL VALSARTAN SODIUM SUSTAINED-RELEASE COMPOSITION, AND PREPARATION METHOD THEREFOR AND USE THEREOF" filed with the China National Intellectual Property Administration on July 12, 2021, which is incorporated herein by reference in its entirety.

### TECHNICAL FIELD

The present disclosure relates to a sacubitril valsartan sodium sustained-release composition, and preparation method therefor and use thereof.

### BACKGROUND

Sacubitril valsartan sodium is suitable for treating chronic heart failure with reduced ejection fraction (HF-REF) in adult patients, reducing the risk of death of cardiovascular diseases and hospitalization due to heart failure. Sacubitril is an enkephalinase inhibitor, while valsartan is an angiotensin II receptor retardant. Sacubitril valsartan sodium can take the place of angiotensin-converting enzyme inhibitors (ACEIs) or angiotensin II receptor blockers (ARBs) in combined use with other heart failure therapies.

Heart failure (HF) is a condition where the heart of a patient cannot provide sufficient blood supply for the body. Symptoms such as dyspnea, fatigue, and fluid retention may occur and gradually worsen, seriously affecting the quality of life. It has been reported that the annual spend on heart failure globally is 108 billion dollars, and a large epidemiological survey in 2003 suggests that the prevalence of heart failure in adults in China was 0.9%, with about 4.5 million people suffering. In another aspect, hypertension is one of the major causes of heart failure and thus a major global public health problem. As reported by Framingham, hypertension causes 75% of heart failures. In China, the rate is 60% to 88.5%, about 6 times that in the population with normal blood pressure. After the occurrence of heart failure, the 5-year death rate is 50% or higher. The incidence of hypertension in China is about 11.88% at present, with an estimated one hundred million hypertension patients.

About half of the heart failure patients have reduced ejection fractions. Existing heart failure therapies can only block harmful effects caused by the activation of the RAAS system (renin-angiotensin-aldosterone system) and are "offensive" strategies rather than "defensive" cardioprotective measures, thus still resulting in a high death rate in patients receiving such therapies. The data show that 50% of the patients will die within 5 years since the diagnosis of heart failure.

The European Society of Cardiology (ESC) Congress 2014 reported a heart failure clinical study (PARADIGM-HF) involving 8442 patients worldwide, of which 353 were Chinese patients. The study shows that patients with heart failure with reduced ejection fraction (HF-REF) receiving a revolutionary heart failure treatment LCZ696 from Novartis had a 20% reduction in death risk and a 21% reduction in hospitalization risk as compared to the enalapril-treated counterpart. The data were published in The New England Journal of Medicine.

Sacubitril valsartan sodium works in a unique and multiplex mode, and can enhance the effect of the cardioprotective neuroendocrine system (the natriuretic peptide system) while inhibiting the harmful effect caused by the activation of the RAAS system. This strategy is both "offensive" and "deffensive". Recent studies show that LCZ696 can be well tolerated and the adverse events can be easily managed, with a low rate of patient withdrawal from the study due to adverse events.

The chemical name of the sacubitril valsartan sodium is octadecasodium hexa(4-{[(1S,3R)-1-([1,1'-biphenyl)-4-ylmethyl)-4-ethoxy-3-methyl-4-oxobutyl]amino}-4-oxobutyric acid)·hexa(N-pentanoyl-N-{[2'-(1H-tetrazol-5-yl)biphenyl-4-yl]methyl}-L-valine)-hydrate (1/15), which has a structural formula as follows:

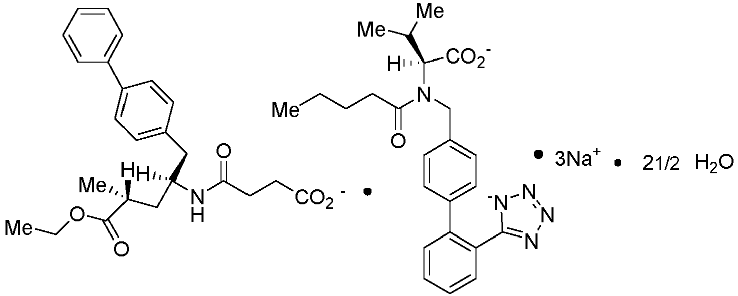

Novartis has developed common sacubitril valsartan sodium immediate-release tablets at a dosage of 50 mg to 200 mg, b.i.d, which possess a high dose and a high administration frequency so far.

Patent reference CN105748420A discloses a method for preparing an LCZ696 sustained-release matrix tablet for treating heart failure. The sustained-release form uses a gel matrix for drug release, featuring a short sustained release period. The active ingredients are completely released in 8 hours, which fails to meet the requirement of a steady release in 24 hours. In addition, the gel-matrix tablet is easily affected by factors such as compression in the gastrointestinal tracts, which may possibly lead to increased drug release rate to different extents, and thus poor sustained-release effect and poor *in vivo*/*in vitro* correlation.

Therefore, to find a dosage form with good sustained-release effects, steady release, and good compliance in patients with chronic heart failure is an urgent task at present.

### SUMMARY

The present disclosure provides a sacubitril valsartan sodium sustained-release composition, wherein the sustained-release composition is a 24-hour sustained-release drug, and the dissolution of sacubitril and valsartan simultaneously meets the following three features:
A) no more than 40% of the active pharmaceutical ingredient is dissolved within 1 hour;
B) 10% to 70% of the active pharmaceutical ingredient is dissolved within 6 hours; and
C) no less than 65% of the active pharmaceutical ingredient is dissolved within 24 hours;
the active pharmaceutical ingredients can be selected from one, two, or more of sacubitril valsartan sodium, or other pharmaceutically acceptable salts, solvates, and hydrates thereof.

According to one embodiment of the present disclosure, in the sacubitril valsartan sodium sustained-release composition, the dissolution of sacubitril simultaneously meets the following three features:
A) no more than 35% of the active pharmaceutical ingredient is dissolved within 1 hour;
B) 10% to 70% of the active pharmaceutical ingredient is dissolved within 6 hours; and
C) no less than 70% of the active pharmaceutical ingredient is dissolved within 24 hours;
the dissolution of valsartan simultaneously meets the following three features:
A) no more than 35% of the active pharmaceutical ingredient is dissolved within 1 hour;
B) 15% to 70% of the active pharmaceutical ingredient is dissolved within 6 hours; and
C) no less than 75% of the active pharmaceutical ingredient is dissolved within 24 hours.

According to one embodiment of the present disclosure, in the sacubitril valsartan sodium sustained-release composition, the dissolution of sacubitril simultaneously meets the following three features:
A) no more than 25% of the active pharmaceutical ingredient is dissolved within 1 hour;
B) 10% to 65% of the active pharmaceutical ingredient is dissolved within 6 hours; and
C) no less than 75% of the active pharmaceutical ingredient is dissolved within 24 hours;
the dissolution of valsartan simultaneously meets the following three features:
A) no more than 30% of the active pharmaceutical ingredient is dissolved within 1 hour;
B) 10% to 65% of the active pharmaceutical ingredient is dissolved within 6 hours; and
C) no less than 75% of the active pharmaceutical ingredient is dissolved within 24 hours.

According to one embodiment of the present disclosure, in the sacubitril valsartan sodium sustained-release composition, the dissolution of sacubitril simultaneously meets the following three features:
A) no more than 25% of the active pharmaceutical ingredient is dissolved within 1 hour;
B) 10% to 65% of the active pharmaceutical ingredient is dissolved within 6 hours; and
C) no less than 80% of the active pharmaceutical ingredient is dissolved within 24 hours;
the dissolution of valsartan simultaneously meets the following three features:
A) no more than 25% of the active pharmaceutical ingredient is dissolved within 1 hour;
B) 15% to 65% of the active pharmaceutical ingredient is dissolved within 6 hours; and
C) no less than 80% of the active pharmaceutical ingredient is dissolved within 24 hours.
preferably, the dissolution of sacubitril and valsartan is a synchronic release.

According to one embodiment of the present disclosure, the sustained-release composition is a 24-hour sustained-release drug, and the dissolution of sacubitril and valsartan simultaneously meets the following three features:
A) no more than 40% of the active pharmaceutical ingredient is dissolved within 2 hours;
B) 20% to 75% of the active pharmaceutical ingredient is dissolved within 8 hours; and
C) no less than 65% of the active pharmaceutical ingredient is dissolved within 24 hours;
the active pharmaceutical ingredients can be selected from one, two, or more of sacubitril valsartan sodium, or other pharmaceutically acceptable salts, solvates, and hydrates thereof.

In one embodiment, in the sacubitril valsartan sodium sustained-release composition, the dissolution of sacubitril simultaneously meets the following three features:
A) no more than 35% of the active pharmaceutical ingredient is dissolved within 2 hours;
B) 20% to 70% of the active pharmaceutical ingredient is dissolved within 8 hours; and
C) no less than 75% of the active pharmaceutical ingredient is dissolved within 24 hours;
the dissolution of valsartan simultaneously meets the following three features:
A) no more than 35% of the active pharmaceutical ingredient is dissolved within 2 hours;
B) 20% to 70% of the active pharmaceutical ingredient is dissolved within 8 hours; and
C) no less than 75% of the active pharmaceutical ingredient is dissolved within 24 hours.

In one embodiment, in the sacubitril valsartan sodium sustained-release composition, the dissolution of sacubitril simultaneously meets the following three features:
A) no more than 25% of the active pharmaceutical ingredient is dissolved within 2 hours;
B) 25% to 65% of the active pharmaceutical ingredient is dissolved within 8 hours; and
C) no less than 75% of the active pharmaceutical ingredient is dissolved within 24 hours;
the dissolution of valsartan simultaneously meets the following three features:
A) no more than 25% of the active pharmaceutical ingredient is dissolved within 2 hours;
B) 25% to 65% of the active pharmaceutical ingredient is dissolved within 8 hours; and
C) no less than 75% of the active pharmaceutical ingredient is dissolved within 24 hours;

In one embodiment, in the sacubitril valsartan sodium sustained-release composition, the dissolution of sacubitril simultaneously meets the following three features:
A) no more than 25% of the active pharmaceutical ingredient is dissolved within 2 hours;
B) 30% to 65% of the active pharmaceutical ingredient is dissolved within 8 hours; and
C) no less than 80% of the active pharmaceutical ingredient is dissolved within 24 hours;
the dissolution of valsartan simultaneously meets the following three features:
A) no more than 25% of the active pharmaceutical ingredient is dissolved within 2 hours;
B) 30% to 65% of the active pharmaceutical ingredient is dissolved within 8 hours; and
C) no less than 80% of the active pharmaceutical ingredient is dissolved within 24 hours;
preferably, the dissolution of sacubitril and valsartan is a synchronic release.

The "dissolution" refers to the cumulative dissolution amount of the active pharmaceutical ingredient, such as the cumulative dissolution amount of sacubitril and the cumulative dissolution amount of valsartan; furthermore, the cumulative dissolution amount is measured in a phosphate buffer at pH 6.8. Those skilled in the art will appreciate that the dissolution of sacubitril valsartan sodium will gradually increase over time.

According to the embodiments of the present disclosure, the sacubitril valsartan sodium sustained-release composition comprises a drug-containing layer core and a coating film, wherein a pore is provided on one side of the drug-containing layer; the drug-containing layer core comprises an active pharmaceutical ingredient and a carrier; the active pharmaceutical ingredient may be selected from one, two, or more of sacubitril valsartan sodium, or other pharmaceutically acceptable salts, solvates, and hydrates thereof; the carrier is, for example, one or more of a tonicity adjusting agent, a sweller, thickener, a glidant, and a lubricant; the coating film is a semipermeable film;
the sustained-release composition optionally further comprises or does not comprise a boosting layer core, and the boosting layer core comprises one or more of a tonicity adjusting agent, a sweller, a colorant and a lubricant.

According to the embodiments of the present disclosure, in the drug-containing layer core, the active pharmaceutical ingredient is preferably sacubitril valsartan sodium.

According to the embodiments of the present disclosure, in the drug-containing layer core, the content of the active pharmaceutical ingredient is 10.00% to 80.00%, for example, 28.25%, 33.93%, 37.67%, 37.70%, 40.39%, 56.50%, 67.87%, or 75.40%, on the weight basis of the drug-containing layer core.

According to the embodiments of the present disclosure, in the drug-containing layer core, the tonicity adjusting agent may be selected from one or more of sodium chloride, potassium chloride, mannitol, sorbitol, sodium sulfate, magnesium sulfate, glucose, fructose, sucrose, and lactose. In one embodiment, the lactose is lactose monohydrate. As an example, the tonicity adjusting agent may be a mixture of sorbitol, lactose monohydrate and sodium chloride, a mixture of sorbitol and sodium chloride, or sodium chloride.

According to the embodiments of the present disclosure, in the drug-containing layer core, the content of the tonicity adjusting agent is 1.00% to 60.00%, for example, 48.75%, 40.34%, 31.50%, 27.50%, 3.00%, or 1.50%, on the weight basis of the drug-containing layer core. According to the embodiments of the present disclosure, in the drug-containing layer core, the thickener (also referred to as suspension or adhesive) is an additive that can increase the viscosity of the dispersion medium to reduce the settling rate of the microparticles or to increase the hydrophilicity of the microparticles, and is selected from, for example, one or more of hydroxypropyl methylcellulose, hydroxypropylcellulose, hydroxyethylcellulose, ethyl cellulose, copovidone, acacia, pregelatinized starch, and polyvinylpyrrolidone. As an example, the thickener is selected from hydroxypropyl methylcellulose, or a mixture of copovidone and hydroxyethylcellulose.

According to the embodiments of the present disclosure, in the drug-containing layer core, the content of the thickener is 10.00% to 70.00%, for example, 21.50%, 10.50%, 21.00%, 15.00%, 13.81%, 57.30%, 34.80%, 25.00%, 53.90%, 56.87%, 19.93%, 18.83%, or 5.41%, on the weight basis of the drug-containing layer core.

According to the embodiments of the present disclosure, in the drug-containing layer core, the sweller (also referred to as promoter) is a substance that can absorb the solvent and expand, and is preferably one or more of polyoxyethylene, carbomer, sodium carboxymethyl starch, crospovidone, and sodium alginate.

According to the embodiments of the present disclosure, in the drug-containing layer core, the content of the sweller is preferably 0 to 70.00%, for example, 48.06%, 24.02%, or 19.93%, on the weight basis of the drug-containing layer core.

According to the embodiments of the present disclosure, the drug-containing layer core may further comprise a colorant.

According to the embodiments of the present disclosure, in the drug-containing layer core, the colorant may be a substance that can achieve the purpose of coloration, and is preferably one or more of iron oxide red, iron oxide yellow, and iron oxide black.

According to the embodiments of the present disclosure, in the drug-containing layer core, the content of the colorant is 0 to 5.00%, for example, 0.10%, on the weight basis of the drug-containing layer core.

According to the embodiments of the present disclosure, in the drug-containing layer core, the glidant may be an auxiliary material that can reduce the friction between particles and improve the flowability of powders or particles, and is preferably one or more of talc, colloidal silicon dioxide, and colloidal silica.

According to the embodiments of the present disclosure, in the drug-containing layer core, the content of the glidant is 0 to 5.00%, for example, 0 to 1.00%, e.g., 0.30%, 0.50%, 1.57%, 1.67%, 3.00%, 3.54%, or 3.57%, on the weight basis of the drug-containing layer core.

According to the embodiments of the present disclosure, in the drug-containing layer core, the lubricant may be a substance having a lubricative effect, and is preferably one or more of a metal stearate salt, stearic acid, talc, a stearate ester, stearyl fumarate, and colloidal silicon dioxide. For example, the metal stearate salt is preferably magnesium stearate and/or calcium stearate. For example, the stearate ester is preferably glyceryl stearate.

According to the embodiments of the present disclosure, in the drug-containing layer core, the content of the lubricant is 0.50% to 60.00%, for example, 1.00% to 30.00%, e.g., 1.00%, 1.20%, 2.00%, 2.14%, 2.33%, or 3.00%, on the weight basis of the drug-containing layer core.

In the drug-containing layer core, the sum of the contents of the active pharmaceutical ingredient and the carrier is 100%.

According to one embodiment of the present disclosure, when the sustained-release composition comprises the boosting layer core, the drug-containing layer core does not comprise the tonicity adjusting agent.

According to the embodiments of the present disclosure, in the boosting layer core, the tonicity adjusting agent may be a substance that adjusts the osmotic pressure, and is preferably one or more of sodium chloride, potassium chloride, mannitol, sorbitol, sodium sulfate, magnesium sulfate, glucose, fructose, sucrose, and lactose, for example, sodium chloride. In one embodiment, the lactose may be lactose monohydrate.

According to the embodiments of the present disclosure, in the boosting layer core, the content of the tonicity adjusting agent is 1.00% to 60.00%, for example, 31.74%, 29.17%, 25.00%, or 5.00%, on the weight basis of the boosting layer core.

According to the embodiments of the present disclosure, in the boosting layer core, the sweller (also referred to as promoter) is a substance that can absorb the solvent and expand, and is preferably one or more of polyvinylpyrrolidone, hydroxyethylcellulose, copovidone, hydroxypropyl methylcellulose, acacia, carbomer, sodium carboxymethyl starch, sodium alginate, and polyoxyethylene.

According to the embodiments of the present disclosure, in the boosting layer core, the content of the sweller is 0 to 90.00%, for example, 0 to 80.00%, e.g., 8.33%, 10.00%, 20.00%, 65.87%, 63.00%, 73.00%, 60.83%, or 82.00%, on the weight basis of the boosting layer core.

According to the embodiments of the present disclosure, in the boosting layer core, the colorant may be a substance that can achieve the purpose of coloration, and is preferably one or more of iron oxide red, iron oxide yellow, and iron oxide black.

According to the embodiments of the present disclosure, in the boosting layer core, the content of the colorant is preferably 0.10% to 10.00%, for example, 1.20%, 1.00%, or 0.83%, on the weight basis of the boosting layer core.

According to the embodiments of the present disclosure, in the boosting layer core, the lubricant may be a conventional substance having a lubricative effect in the art, and is preferably one or more of a metal stearate salt, stearic acid, talc, a stearate ester, stearyl fumarate, and colloidal silicon dioxide. For example, the metal stearate salt is preferably magnesium stearate and/or calcium stearate. For example, the stearate ester is preferably glyceryl stearate.

According to the embodiments of the present disclosure, in the boosting layer core, the content of the lubricant is preferably 0.10% to 10.00%, for example, 2.00%, 1.20%, 1.00%, or 0.84%, on the weight basis of the boosting layer core.

In the boosting layer core, the sum of the contents of the components is 100%.

According to the embodiments of the present disclosure, the semipermeable film comprises one or more of a film-forming material, a porogen, and a plasticizer.

According to the embodiments of the present disclosure, in the coating film, the film-forming material may be a material that can be dispersed on a solid surface and cured to form a film, and is preferably one or more of cellulose acetate, ethylcellulose, cellulose acetate phthalate, polyvinyl acetate, acrylic resin, and methacrylic resin.

According to the embodiments of the present disclosure, in the coating film, the porogen may be a substance that can form pores in a material after contact with water, and is preferably one or more of polyethylene glycol, hydroxypropylcellulose, povidone, and polyvinyl alcohol. According to the embodiments of the present disclosure, in the coating film, the plasticizer may be a substance that can increase the plasticity of a polymer, and is preferably one or more of glyceryl triacetate, glyceryl citrate, a glyceride, dibutyl phthalate, and diethyl phthalate.

In one embodiment, the polyethylene glycol is polyethylene glycol 4000.

In one embodiment, the coating film comprises cellulose acetate and polyethylene glycol 4000.

In one embodiment, the coating film may also be a fully formulated cellulose acetate coating premix (Opadry^{®} CA) provided by Colorcon.

According to the embodiments of the present disclosure, when the sacubitril valsartan sodium sustained-release composition is tested for dissolution by method II in the USP in 900 mL of phosphate buffer (pH 6.8) at 50 rpm, the 24-hour cumulative release rate of sacubitril and valsartan can be 85% or greater.

According to exemplary embodiments of the present disclosure, the drug-containing layer core may be any one of the following formulas:
formula I: 28.25% of sacubitril valsartan sodium, 25.00% of sorbitol, 21.25% of lactose monohydrate, 2.50% of sodium chloride, 21.50% of hydroxypropyl methylcellulose, 0.50% of colloidal silica, and 1.00% of magnesium stearate, on the weight basis of the drug-containing layer core;
formula II: 56.50% of sacubitril valsartan sodium, 20.00% of sorbitol, 10.00% of lactose monohydrate, 1.50% of sodium chloride, 10.50% of hydroxypropyl methylcellulose, 0.50% of colloidal silica, and 1.00% of magnesium stearate, on the weight basis of the drug-containing layer core;
formula III: 37.67% of sacubitril valsartan sodium, 36.66% of sorbitol, 3.67% of sodium chloride, 21.00% of hydroxypropyl methylcellulose, and 1.00% of magnesium stearate, on the weight basis of the drug-containing layer core;
formula IV: 56.50% of sacubitril valsartan sodium, 25.00% of sorbitol, 2.50% of sodium chloride, 15.00% of hydroxypropyl methylcellulose, and 1.00% of magnesium stearate, on the weight basis of the drug-containing layer core;
formula V: 33.93% of sacubitril valsartan sodium, 48.06% of polyoxyethylene, 3.00% of sodium chloride, 13.81% of hydroxypropyl methylcellulose, and 1.20% of magnesium stearate, on the weight basis of the drug-containing layer core;
formula VI: 67.87% of sacubitril valsartan sodium, 24.02% of polyoxyethylene, 1.5% of sodium chloride, 5.41% of hydroxypropyl methylcellulose, and 1.20% of magnesium stearate, on the weight basis of the drug-containing layer core;
formula VII: 37.70% of sacubitril valsartan sodium, 38.63% of copovidone, 18.67% of hydroxyethylcellulose, 2.33% of talc, 0.67% of colloidal silica, and 2.00% of magnesium stearate, on the weight basis of the drug-containing layer core;
formula VIII: 75.40% of sacubitril valsartan sodium, 11.60% of copovidone, 8.33% of hydroxyethylcellulose, 1.00% of talc, 0.67% of colloidal silica, and 3.00% of magnesium stearate, on the weight basis of the drug-containing layer core;
formula IX: 40.39% of sacubitril valsartan sodium, 26.90% of copovidone, 27.00% of hydroxyethylcellulose, 2.86% of talc, 0.71% of colloidal silica, and 2.14% of magnesium stearate, on the weight basis of the drug-containing layer core;
formula X: 75.40% of sacubitril valsartan sodium, 19.93% of polyoxyethylene, 1.00% of talc, 0.67% of colloidal silica, and 3.00% of magnesium stearate, on the weight basis of the drug-containing layer core;
formula XI: 37.70% of sacubitril valsartan sodium, 28.20% of copovidone, 28.66% of hydroxyethylcellulose, 2.67% of talc, 0.67% of colloidal silica, 2.00% of magnesium stearate, and 0.10% of iron oxide yellow, on the weight basis of the drug-containing layer core; and
formula XII: 75.40% of sacubitril valsartan sodium, 6.83% of copovidone, 12.00% of hydroxyethylcellulose, 2.67% of talc, 0.67% of colloidal silica, 2.33% of magnesium stearate, and 0.10% of iron oxide yellow, on the weight basis of the drug-containing layer core.

According to an exemplary embodiment of the present disclosure, the boosting layer core is any one of the following formulas:
formula I: 65.86% of polyoxyethylene, 31.74% of sodium chloride, 1.20% of iron oxide red, and 1.20% of magnesium stearate, on the weight basis of the boosting layer core;
formula II: 73.00% of polyoxyethylene, 20.00% of copovidone, 1.00% of iron oxide red, 5.00% of sodium chloride, and 1.00% of magnesium stearate, on the weight basis of the boosting layer core;
formula III: 60.83% of polyoxyethylene, 8.33% of copovidone, 29.17% of sodium chloride, 0.83% of iron oxide red, and 0.84% of magnesium stearate, on the weight basis of the boosting layer core;
formula IV: 82.00% of polyoxyethylene, 10.00% of copovidone, 5.00% of sodium chloride, 1.00% of iron oxide red, and 2.00% of magnesium stearate, on the weight basis of the boosting layer core; and
formula V: 63.00% of polyoxyethylene, 10.00% of copovidone, 25.00% of sodium chloride, 1.00% of iron oxide red, and 1.00% of magnesium stearate, on the weight basis of the boosting layer core.

According to exemplary embodiments of the present disclosure, the coating is any one of the following compositions:
coating I: 9.00% of cellulose acetate and 1.00% of polyethylene glycol 4000, on the weight basis of the tablet core;
the coating solvent is acetone and water, for example, in an acetone-to-water mass ratio of 9: 1;
the solid content of the coating solution is 5%, on the weight basis of the coating solution;
coating II: 7.20% of cellulose acetate and 0.80% of polyethylene glycol 4000, on the weight basis of the tablet core;
the coating solvent is acetone and water, for example, in an acetone-to-water mass ratio of 9: 1;
the solid content of the coating solution is 5%, on the weight basis of the coating solution; and
coating III: 6% of cellulose acetate and 3% of polyethylene glycol 4000, on the weight basis of the tablet core;
the coating solvent is acetone and water, for example, in an acetone-to-water mass ratio of 9: 1; the solid content of the coating solution is 5%, on the weight basis of the coating solution.

In one embodiment, the coating solution is a mixed solution of the coating solvent, cellulose acetate, and polyethylene glycol 4000.

The tablet core consists of the drug-containing layer core and the boosting layer core; when the boosting layer core is absent, the tablet core is the drug-containing layer core.

The present disclosure further provides a method for preparing the sustained-release composition described above, comprising the following steps:
mixing the components of the drug-containing layer core, and tabletting to give the drug-containing layer core (the preparation process may be direct powder tabletting, dry granulation, wet granulation, etc.); and forming a pore on one side of the drug-containing layer after the drug-containing layer core is coated (by a semipermeable film coating) to give the sacubitril valsartan sodium sustained-release composition.

In one embodiment, the method comprises the following specific steps:
step 1): sieving and mixing the other components of the drug-containing layer core than the lubricant, and then mixing with the lubricant to give a drug-containing layer mixture;
or alternatively, sieving and mixing the other components of the drug-containing layer core than the lubricant and the glidant, and then mixing with the lubricant and the glidant to give a drug-containing layer mixture;
step 2): mixing the drug-containing layer mixture obtained in step 1 with the lubricant, and tabletting to give the drug-containing core;
step 3): coating and aging the drug-containing layer core obtained in step 2) to give a coated tablet; and
step 4): forming a pore on the coated tablet obtained in step 3) to give the sacubitril valsartan sodium sustained-release tablet.

According to the embodiments of the present disclosure, in step 1), the sieving may be performed with a 60-mesh sieve or a 40-mesh sieve.

According to the embodiments of the present disclosure, in step 1), the mixing may be performed in a mixer, e.g., a mixing tank. The speed of the mixing may be 18 r/min and the period of the mixing may be 1 minute to 2 hours, e.g., 20 minutes.

According to the embodiments of the present disclosure, in step 2), the rigidity of the drug-containing core is 100 N to 160 N.

According to the embodiments of the present disclosure, in step 3), the temperature of the coating is preferably 25 °C to 45 °C, e.g., 30 °C.

According to the embodiments of the present disclosure, in step 3), the coating preferably causes a weight gain of 6% to 20%, wherein the percentage is (weight of the coated sacubitril valsartan sodium tablet - weight of the drug-containing layer core)/weight of the drug-containing layer core × 100%.

According to the embodiments of the present disclosure, in step 3), the temperature of the aging is preferably 20 °C to 60 °C, e.g., 45 °C.

According to the embodiments of the present disclosure, in step 3), the period of the aging is preferably 10 hours to 30 hours, e.g., 24 hours.

According to the embodiments of the present disclosure, in step 4), the pore formation may be performed at a central position on one side of the drug-containing layer. The size of the pore may be 0.4 mm to 0.7 mm.

The present disclosure further provides a method for preparing a sustained-release composition, comprising: separately mixing components of a drug-containing layer core and components of a boosting layer core, and tabletting to give a double-layer core (the preparation process of the mixture powders may be direct powder tabletting, dry granulation, wet granulation, etc.); and forming a pore on one side of the drug-containing layer after the double-layer core is coated (by a semipermeable film coating) to give the sacubitril valsartan sodium sustained-release composition.

In one embodiment, the method comprises the following specific steps:
step A1): sieving and mixing the other components of a drug-containing layer core than a lubricant, and then mixing with the lubricant to give a drug-containing layer mixture;
step A2): sieving and mixing the other components of a boosting layer core than a lubricant, and then mixing with the lubricant to give a boosting layer mixture;
step A3): tabletting the drug-containing layer mixture obtained in step A1) and the boosting layer mixture obtained in step A2) to give a drug-containing core;
step A4): coating and aging the drug-containing core obtained in step A3) to give a coated tablet; and
step A5): forming a pore on the side of the drug-containing layer of the coated tablet obtained in step A4) to give the sacubitril valsartan sodium sustained-release tablet.

According to the embodiments of the present disclosure, in steps A1) and A2), the sieving may be performed with a 40-mesh or 60-mesh sieve.

According to the embodiments of the present disclosure, in steps A1) and A2), the mixing may be performed in a mixer, e.g., a mixing tank. The speed of the mixing may be 18 r/min and the period of the mixing may be 1 minute to 2 hours, e.g., 5 minutes, 10 minutes, or 15 minutes.

According to the embodiments of the present disclosure, in step A4), the temperature of the coating is preferably 25 °C to 45 °C, e.g., 30 °C.

According to the embodiments of the present disclosure, in step A4), the coating preferably causes a weight gain of 6% to 20%, wherein the percentage is (weight of the coated sacubitril valsartan sodium tablet - weight of the drug-containing core)/weight of the drug-containing core × 100%.

According to the embodiments of the present disclosure, in step A4), the temperature of the aging is preferably 20 °C to 60 °C, e.g., 45 °C.

According to the embodiments of the present disclosure, in step A4), the period of the aging is preferably 10 hours to 30 hours, e.g., 24 hours.

According to the embodiments of the present disclosure, in step A5), the pore formation may be performed at a central position on the drug-containing surface. For example, the size of the pore may be 0.4 mm to 0.7 mm.

The present disclosure further provides a sacubitril valsartan sodium sustained-release preparation, comprising the sacubitril valsartan sodium sustained-release composition.

According to the embodiments of the present disclosure, the sacubitril valsartan sodium sustained-release preparation may be a sustained-release tablet, a sustained-release pellet, a sustained-release capsule, etc.

The present disclosure further provides use of the sacubitril valsartan sustained-release composition or the sacubitril valsartan sustained-release preparation for manufacturing a medicament for treating and/or preventing chronic heart failure with reduced ejection fraction.

The present disclosure further provides a method for treating and/or preventing chronic heart failure with reduced ejection fraction, comprising administering to a patient in need an effective dose of the sacubitril valsartan sustained-release preparation.

The reagents and starting materials used in the present disclosure are commercially available.

Beneficial Effects of Present Disclosure:
The present disclosure provides a sacubitril valsartan sodium sustained-release composition different from the prior art, featuring good sustained-release effects, stable release and good compliance in patients with chronic heart failure, and a method for preparing same, and use thereof. The sacubitril valsartan sodium sustained-release composition of the present disclosure features drug release performance that is not influenced by factors such as medium pH, gastrointestinal motility and food, and good *in vivo*/*in vitro* correlation. Sacubitril sodium and valsartan sodium can be synchronously and smoothly released within 24 hours, leading to a better therapeutic effect. Compared with common immediate-release preparations, the preparation has the advantages that: (1) the frequency of administration is reduced and the efficacy is improved via the sustained release, resulting in improved patient compliance; (2) for elders, children, and other populations with dysphagia, the compliance is improved; (3) the treatment interruption can be avoided, and the disease relapse and malignant complication progression are prevented; and (4) the preparation is administered once daily, and the proper specification can be selected according to the clinical conditions, featuring reduced administration frequency, convenience of use, and mild adverse effects.

### BRIEF DESCRIPTION OF THE DRAWING

FIG. 1 shows the dissolution profile of sacubitril and valsartan in a phosphate buffer at pH 6.8 according to Example 1-1;
FIG. 2 shows the dissolution profile of sacubitril and valsartan in a phosphate buffer at pH 6.8 according to Example I-2;
FIG. 3 shows the dissolution profile of sacubitril and valsartan in a phosphate buffer at pH 6.8 according to Example I-3;
FIG. 4 shows the dissolution profile of sacubitril and valsartan in a phosphate buffer at pH 6.8 according to Example I-4;
FIG. 5 shows the dissolution profile of sacubitril and valsartan in a phosphate buffer at pH 6.8 according to Example I-5;
FIG. 6 shows the dissolution profile of sacubitril and valsartan in a phosphate buffer at pH 6.8 according to Example I-6;
FIG. 7 shows the dissolution profile of sacubitril and valsartan in a phosphate buffer at pH 6.8 according to Example II-1;
FIG. 8 shows the dissolution profile of sacubitril and valsartan in a phosphate buffer at pH 6.8 according to Example II-2;
FIG. 9 shows the dissolution profile of sacubitril and valsartan in a phosphate buffer at pH 6.8 according to Example II-3;
FIG. 10 shows the dissolution profile of sacubitril and valsartan in an acetate buffer at pH 4.5 according to Example II-3;
FIG. 11 shows the dissolution profile of sacubitril and valsartan in an aqueous solution according to Example II-3;
FIG. 12 shows the dissolution profile of sacubitril and valsartan in a phosphate buffer at pH 6.8 according to Example II-4;
FIG. 13 shows the dissolution profile of sacubitril and valsartan in a phosphate buffer at pH 6.8 according to Example II-5;
FIG. 14 shows the dissolution profile of sacubitril and valsartan in a phosphate buffer at pH 6.8 according to Example II-6;
FIG. 15 shows the dissolution profile of sacubitril and valsartan in an acetate buffer at pH 4.5 according to Example II-6;
FIG. 16 shows the dissolution profile of sacubitril and valsartan in an aqueous solution according to Example II-6;
FIG. 17 shows the dissolution profile of sacubitril and valsartan in a phosphate buffer at pH 6.8 according to Example II-7; and
FIG. 18 shows the dissolution profile of sacubitril and valsartan in a phosphate buffer at pH 6.8 according to Example II-8.

### DETAILED DESCRIPTION

The embodiments of the present disclosure will be further described in detail with reference to the following specific examples. It will be appreciated that the following examples are merely exemplary illustrations and explanations of the present disclosure, and should not be construed as limiting the protection scope of the present disclosure. All techniques implemented based on the content of the present disclosure described above are included within the protection scope of the present disclosure.

Unless otherwise stated, the starting materials and reagents used in the following examples are all commercially available products, or can be prepared by using known methods.

Dissolution test: the sacubitril valsartan sodium sustained-release composition was tested for dissolution by method II in the USP in 900 mL of phosphate buffer (pH 6.8) at 50 rpm.

### Examples 1-1 and I-2

| Serial number | Example I-1 | | Example I-2 | |
|---|---|---|---|---|
| Name | Amount per tablet (mg) | Proportion (%) | Amount per tablet (mg) | Proportion (%) |
| **Core** | | | | |
| Sacubitril valsartan sodium | 113.00 | 28.25 | 226.00 | 56.50 |
| Sorbitol | 100.00 | 25.00 | 80.00 | 20.00 |
| Lactose monohydrate | 85.00 | 21.25 | 40.00 | 10.00 |
| Sodium chloride | 10.00 | 2.50 | 6.00 | 1.50 |
| Hydroxypropyl methylcellulose | 86.00 | 21.50 | 42.00 | 10.50 |
| Colloidal silica | 2.00 | 0.50 | 2.00 | 0.50 |
| Magnesium stearate | 4.00 | 1.00 | 4.00 | 1.00 |
| Weight of core | 400.00 | 100.00 | 400.00 | 100.00 |

| **Coating** | | | | |
|---|---|---|---|---|
| Cellulose acetate | 36.00 | 9.00 | 36.00 | 9.00 |
| Polyethylene glycol 4000 | 4.00 | 1.00 | 4.00 | 1.00 |
| Acetone | 684.00 | / | 684.00 | / |
| Purified water | 76.00 | / | 76.00 | / |

### Preparation procedure:

Sacubitril valsartan sodium described above was sieved with a 60-mesh sieve; sorbitol and colloidal silica were sieved with a 40-mesh sieve to remove the lumps.

Step 1: sacubitril valsartan sodium, sorbitol, lactose monohydrate, sodium chloride, and hydroxypropyl methylcellulose in the formula described above were added into a mixing tank and mixed at a mixing speed of 18 r/min for 20 min to give a drug-containing layer mixture. Step 2: The drug-containing layer mixture obtained in Step 1 was mixed with colloidal silica at a mixing speed of 18 r/min for 5 min, and then mixed with magnesium stearate at a mixing speed of 18 r/min for 5 min. Then the mixture was loaded on a rotary tablet press and tabletted into shallow-convex round drug-containing cores (400 mg/tablet) with a rigidity of 100 N to 160 N.

### Step 3:

A sustained-release coating solution was prepared according to the following steps: Purified water was added to a beaker and stirred by a magnetic stirrer with acetone added during the stirring. The mixture was uniformly stirred for another 5 minutes after the addition of acetone was completed. Opadry^{®} CA was added slowly to the mixture and the mixture was continuously stirred for 30 to 120 minutes for a complete dissolution to give the sustained-release coating solution.

The drug-containing cores obtained in Step 2 were coated with the sustained-release coating solution described above. At a coating pan rotation speed of 8 rpm to 15 rpm, an inlet air volume of 30 m³/h to 120 m³/h, a tablet bed temperature of 25 °C to 35 °C, and a spraying speed of 5 rpm to 20 rpm, the solution was sprayed for coating until the coating weight gains were 10.3% and 10.1% respectively. The tablet cores were dried at a tablet bed temperature of 30 °C to 35 °C for 5 to 10 minutes, dried in an air blast dryer at 45 °C, and aged for 24 hours to give the coated tablets.

Step 4: On the coated tablets obtained in Step 3, a pore was formed by laser with a size of 0.4 to 0.7 mm to give the sacubitril valsartan sodium sustained-release tablets.

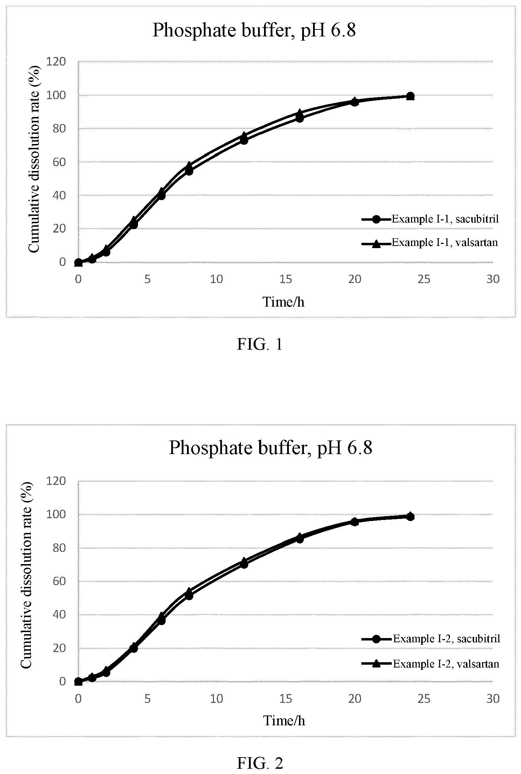

The dissolution data of the sacubitril valsartan sodium sustained-release tablets prepared by the above steps are shown in Table 1 and FIGs. 1 and 2.

**Table 1. Dissolution rates of sacubitril valsartan sodium sustained-release tablets prepared in Examples 1-1 and I-2**

| Serial number | Example I-1 | | Example I-2 | |
|---|---|---|---|---|
| Active ingredient | Sacubitril | Valsartan | Sacubitril | Valsartan |
| Time (h) | Cumulative dissolution rate (%) | | | |
| 1 | 1.9 | 2.9 | 2.1 | 3.0 |
| 2 | 6.0 | 8.2 | 5.3 | 6.9 |
| 4 | 22.3 | 25.3 | 19.8 | 21.3 |
| 6 | 39.8 | 42.6 | 36.4 | 39.3 |
| 8 | 54.5 | 58.1 | 51.2 | 54.1 |
| 12 | 72.8 | 75.9 | 70.2 | 72.3 |
| 16 | 86.1 | 89.5 | 85.3 | 86.7 |
| 20 | 95.9 | 96.7 | 95.3 | 96.0 |
| 24 | 99.7 | 99.6 | 98.6 | 99.3 |

### Examples I-3 and I-4

| Serial number | Example I-3 | | Example I-4 | |
|---|---|---|---|---|
| Name | Amount per tablet (mg) | Proportion (%) | Amount per tablet (mg) | Proportion (%) |
| **Core (internal)** | | | | |
| Sacubitril valsartan sodium | 113.00 | 37.67 | 226.00 | 56.50 |
| Sorbitol | 110.00 | 36.66 | 100.00 | 25.00 |
| Sodium chloride | 11.00 | 3.67 | 10.00 | 2.50 |
| Hydroxypropyl methylcellulose | 63.00 | 21.00 | 60.00 | 15.00 |
| Magnesium stearate | 1.50 | 0.50 | 2.00 | 0.50 |

| **Core (external)** | | | | |
|---|---|---|---|---|
| Magnesium stearate | 1.50 | 0.50 | 2.00 | 0.50 |
| Weight of core | 300.00 | 100.00 | 400.00 | 100.00 |

| **Coating** | | | | |
|---|---|---|---|---|
| Cellulose acetate | 21.60 | 7.20 | 28.80 | 7.20 |
| Polyethylene glycol 4000 | 2.40 | 0.80 | 3.20 | 0.80 |
| Acetone | 410.40 | / | 547.20 | / |
| Purified water | 45.60 | / | 60.80 | / |

### Preparation procedure:

Sacubitril valsartan sodium described above was sieved with a 60-mesh sieve; sorbitol was sieved with a 40-mesh sieve to remove the lumps.

Step 1: sacubitril valsartan sodium, sorbitol, sodium chloride, and hydroxypropyl methylcellulose in the formula described above were added into a mixing tank and mixed at a mixing speed of 18 r/min for 20 min before internal magnesium stearate was added; the mixture was mixed at a mixing speed of 18 r/min for 5 min to give a drug-containing layer mixture.

Step 2: The drug-containing layer mixture obtained in Step 1 was loaded on a dry granulator. The parameters of the granulator were adjusted such that the mixture was tabletted into large tablets with a certain rigidity. The tablets were sized by using a 1.0-mm sieve. Magnesium stearate was added and the mixture was mixed at a mixing speed of 18 r/min for 5 min, loaded on a rotary tablet press, and tabletted into shallow-convex round drug-containing cores.

### Step 3:

A sustained-release coating solution was prepared according to the following steps: Purified water was added to a beaker and stirred by a magnetic stirrer with acetone added during the stirring. The mixture was uniformly stirred for another 5 minutes after the addition of acetone was completed. Opadry^{®} CA was added slowly to the mixture and the mixture was continuously stirred for 30 to 120 minutes for a complete dissolution to give the sustained-release coating solution.

The drug-containing cores obtained in Step 2 were coated with the sustained-release coating solution described above. At a coating pan rotation speed of 8 rpm to 15 rpm, an inlet air volume of 30 m³/h to 120 m³/h, a tablet bed temperature of 25 °C to 35 °C, and a spraying speed of 5 rpm to 20 rpm, the solution was sprayed for coating until the coating weight gains were 7.9% and 8.2% respectively. The tablet cores were dried at a tablet bed temperature of 30 °C to 35 °C for 5 to 10 minutes, dried in an air blast dryer at 45 °C, and aged for 24 hours to give the coated tablets.

Step 4: On the coated tablets obtained in Step 3, a pore was formed by laser with a size of 0.4 to 0.7 mm to give the sacubitril valsartan sodium sustained-release tablets.

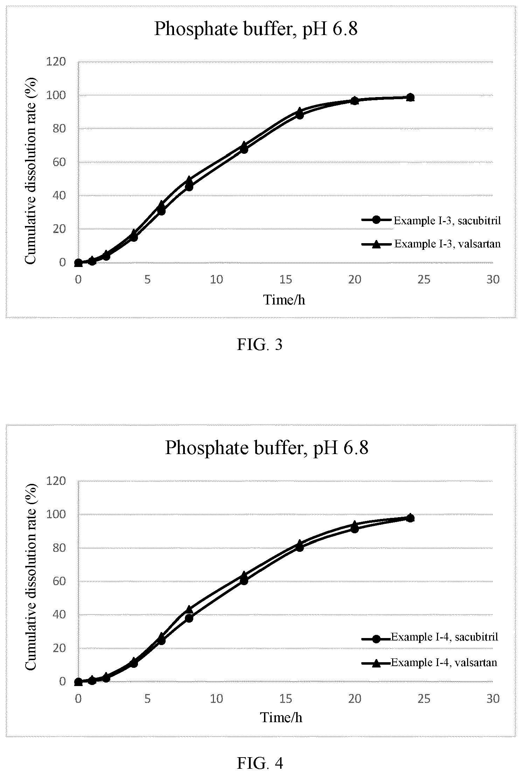

The dissolution data of the sacubitril valsartan sodium sustained-release tablets prepared by the above steps are shown in Table 2 and FIGs. 3 and 4.

**Table 2. Dissolution rates of sacubitril valsartan sodium sustained-release tablets prepared in Examples I-3 and I-4**

| Serial number | Example I-3 | | Example I-4 | |
|---|---|---|---|---|
| Active ingredient | Sacubitril | Valsartan | Sacubitril | Valsartan |
| Time (h) | Cumulative dissolution rate (%) | | | |
| 1 | 0.9 | 1.6 | 0.6 | 1.3 |
| 2 | 3.8 | 5.3 | 2.1 | 3.2 |
| 4 | 15.2 | 17.6 | 10.8 | 12.3 |
| 6 | 30.6 | 34.9 | 24.5 | 27.2 |
| 8 | 45.2 | 49.7 | 37.9 | 43.2 |
| 12 | 67.6 | 70.2 | 60.3 | 63.8 |
| 16 | 88.1 | 90.6 | 80.2 | 82.6 |
| 20 | 96.7 | 97.2 | 91.2 | 94.0 |
| 24 | 98.9 | 99.1 | 97.7 | 98.3 |

### Examples I-5 and I-6

| Serial number | Example I-5 | | Example I-6 | |
|---|---|---|---|---|
| Name | Amount per tablet (mg) | Proportion (%) | Amount per tablet (mg) | Proportion (%) |
| **Drug-containing layer** | | | | |
| Sacubitril valsartan sodium | 113.00 | 33.93 | 226.00 | 67.87 |
| Polyoxyethylene | 160.00 | 48.05 | 80.00 | 24.02 |
| Sodium chloride | 10.00 | 3.00 | 5.00 | 1.50 |
| Hydroxypropyl methylcellulose | 46.00 | 13.81 | 18.00 | 5.41 |
| Magnesium stearate | 4.00 | 1.20 | 4.00 | 1.20 |
| Total | 333.00 | 100.00 | 333.00 | 100.00 |

| **Boosting layer** | | | | |
|---|---|---|---|---|
| Polyoxyethylene | 110.00 | 65.86 | 110.00 | 65.86 |
| Sodium chloride | 53.00 | 31.74 | 53.00 | 31.74 |
| Iron oxide red | 2.00 | 1.20 | 2.00 | 1.20 |
| Magnesium stearate | 2.00 | 1.20 | 2.00 | 1.20 |
| Total | 167.00 | 100.00 | 167.00 | 100.00 |
| Weight of core | 500.00 | / | 500.00 | / |

| **Coating** | | | | |
|---|---|---|---|---|
| Cellulose acetate | 36.00 | 9.00 | 36.00 | 9.00 |
| Polyethylene glycol 4000 | 4.00 | 1.00 | 4.00 | 1.00 |
| Acetone | 684.00 | / | 684.00 | / |
| Purified water | 76.00 | / | 76.00 | / |

### Preparation procedure:

Sacubitril valsartan sodium described above was sieved with a 60-mesh sieve.

Step 1: sacubitril valsartan sodium, polyoxyethylene, sodium chloride, and hydroxypropyl methylcellulose in the drug-containing layer of the formula described above were added into a mixing tank and mixed at a mixing speed of 18 r/min for 20 min before magnesium stearate in the drug-containing layer was added; the mixture was mixed at a mixing speed of 18 r/min for 5 min to give a drug-containing layer mixture.

Step 2: Polyoxyethylene, sodium chloride, and iron oxide red in the boosting layer of the formula described above were added into a mixing tank and mixed at a mixing speed of 18 r/min for 10 min before magnesium stearate in the boosting layer was added; the mixture was mixed at a mixing speed of 18 r/min for 5 min to give a boosting layer mixture.

Step 3: The drug-containing layer mixture obtained in Step 1 and the boosting layer mixture obtained in Step 2 were loaded on a rotary tablet press. The parameters of the press were adjusted such that the weight of the drug-containing layer was 333 mg, the weight of the boosting layer was 167 mg, the total weight of the tablet was 500 mg, and the rigidity was 100 N to 180 N. The mixtures were tabletted into shallow-convex round drug-containing cores.

### Step 4:

A sustained-release coating solution was prepared according to the following steps: Purified water was added to a beaker and stirred by a magnetic stirrer with acetone added during the stirring. The mixture was uniformly stirred for another 5 minutes after the addition of acetone was completed. Opadry^{®} CA was added slowly to the mixture and the mixture was continuously stirred for 30 to 90 minutes for a complete dissolution to give the sustained-release coating solution.

The drug-containing cores obtained in Step 3 were coated with the sustained-release coating solution described above. At a rotation speed of 8 rpm to 15 rpm, a tablet bed temperature of 25 °C to 35 °C, and a spraying speed of 5 rpm to 20 rpm, the solution was sprayed for coating until the coating weight gains were 8.2% and 8.0% respectively. The tablet cores were dried at a tablet bed temperature of 30 °C to 35 °C for 5 to 10 minutes, dried in an air blast dryer at 45 °C, and aged for 24 hours to give the coated tablets.

Step 5: On the drug-containing layer (white) of the coated tablets obtained in Step 4, a pore was formed by laser with a size of 0.4 to 0.7 mm to give the sacubitril valsartan sodium sustained-release tablets.

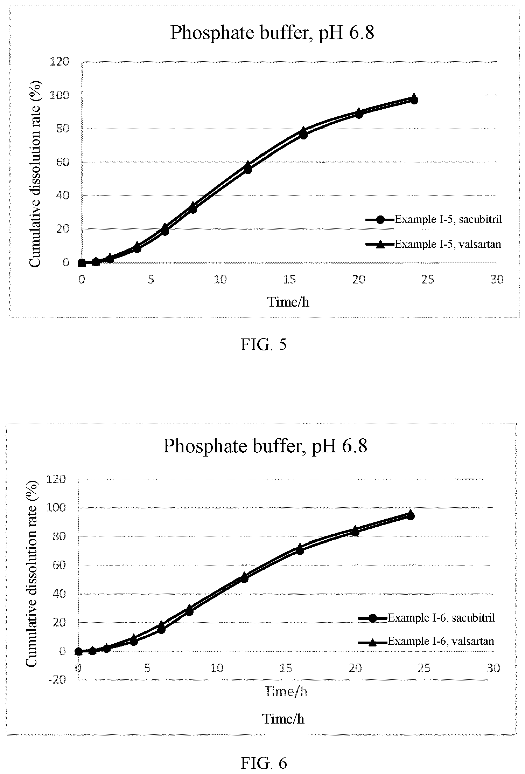

The dissolution data of the sacubitril valsartan sodium sustained-release tablets prepared by the above steps are shown in Table 1 and FIGs. 5 and 6.

**Table 3. Dissolution rates of sacubitril valsartan sodium sustained-release tablets prepared in Examples I-5 and I-6**

| Serial number | Example I-5 | | Example I-6 | |
|---|---|---|---|---|
| Active ingredient | Sacubitril | Valsartan | Sacubitril | Valsartan |
| Time (h) | Cumulative dissolution rate (%) | | | |
| 1 | 0.3 | 0.9 | 0.2 | 1.0 |
| 2 | 2.1 | 3.1 | 1.9 | 2.9 |
| 4 | 8.3 | 10.2 | 7.0 | 9.6 |
| 6 | 18.7 | 21.3 | 15.3 | 18.9 |
| 8 | 31.8 | 34.1 | 27.6 | 30.1 |
| 12 | 55.6 | 58.6 | 50.6 | 52.7 |
| 16 | 76.2 | 79.0 | 70.3 | 72.8 |
| 20 | 88.6 | 90.3 | 83.1 | 85.3 |
| 24 | 97.2 | 98.9 | 94.2 | 96.3 |

### Example II-1

| Name | Amount per tablet (mg) | Proportion (%) |
|---|---|---|
| **Drug-containing layer** | | |
| Sacubitril valsartan sodium | 113.10 | 37.70 |
| Colloidal silica | 2.00 | 0.67 |
| Copovidone | 115.9 | 38.63 |
| Hydroxyethylcellulose | 56.00 | 18.67 |
| Talc | 7.00 | 2.33 |
| Magnesium stearate | 6.00 | 2.00 |
| Total | 300.00 | 100.00 |

| **Boosting layer** | | |
|---|---|---|
| Polyoxyethylene | 63.00 | 63.00 |
| Copovidone | 10.00 | 10.00 |
| Iron oxide red | 1.00 | 1.00 |
| Sodium chloride | 25.00 | 25.00 |
| Magnesium stearate | 1.00 | 1.00 |
| Total | 100.00 | 100.00 |
| Weight of core | 400.00 | / |

| **Coating** | | |
|---|---|---|
| Cellulose acetate | 36.00 | 9.00 |
| Polyethylene glycol 4000 | 4.00 | 1.00 |
| Acetone | 684.00 | / |
| Purified water | 76.00 | / |

### Preparation procedure:

Sacubitril valsartan sodium described above was sieved with a 60-mesh sieve.

Step 1: sacubitril valsartan sodium, copovidone, hydroxyethylcellulose, colloidal silica, and talc in the drug-containing layer of the formula described above were added into a mixing tank and mixed at a mixing speed of 18 r/min for 20 min before the mixture was sized by dry granulation and magnesium stearate in the drug-containing layer was added; the mixture was mixed at a mixing speed of 18 r/min for 5 min to give a drug-containing layer mixture.

Step 2: Polyoxyethylene, sodium chloride, copovidone, and iron oxide red in the boosting layer of the formula described above were added into a mixing tank and mixed at a mixing speed of 18 r/min for 10 min before magnesium stearate in the boosting layer was added; the mixture was mixed at a mixing speed of 18 r/min for 5 min to give a boosting layer mixture.

Step 3: The drug-containing layer mixture obtained in Step 1 and the boosting layer mixture obtained in Step 2 were loaded on a rotary tablet press. The parameters of the press were adjusted such that the weight of the drug-containing layer was 300 mg, the weight of the boosting layer was 100 mg, the total weight of the tablet was 400 mg, and the rigidity was 100 N to 180 N. The mixtures were tabletted into shallow-convex round drug-containing cores.

### Step 4:

A sustained-release coating solution was prepared according to the following steps: Purified water was added to a beaker and stirred by a magnetic stirrer with acetone added during the stirring. The mixture was uniformly stirred for another 5 minutes after the addition of acetone was completed. Opadry^{®} CA was added slowly to the mixture and the mixture was continuously stirred for 30 to 90 minutes for a complete dissolution to give the sustained-release coating solution.

The drug-containing cores obtained in Step 3 were coated with the sustained-release coating solution described above. At a rotation speed of 8 rpm to 15 rpm, a tablet bed temperature of 25 °C to 35 °C, and a spraying speed of 5 rpm to 20 rpm, the solution was sprayed for coating until the coating weight gain was 8.0% to 11.0%. The tablet cores were dried at a tablet bed temperature of 30 °C to 35 °C for 5 to 10 minutes, dried in an air blast dryer at 45 °C, and aged for 24 hours to give the coated tablets.

Step 5: On the drug-containing layer (white) of the coated tablets obtained in Step 4, a pore was formed by laser with a size of 0.4 to 0.7 mm to give the sacubitril valsartan sodium sustained-release tablets.

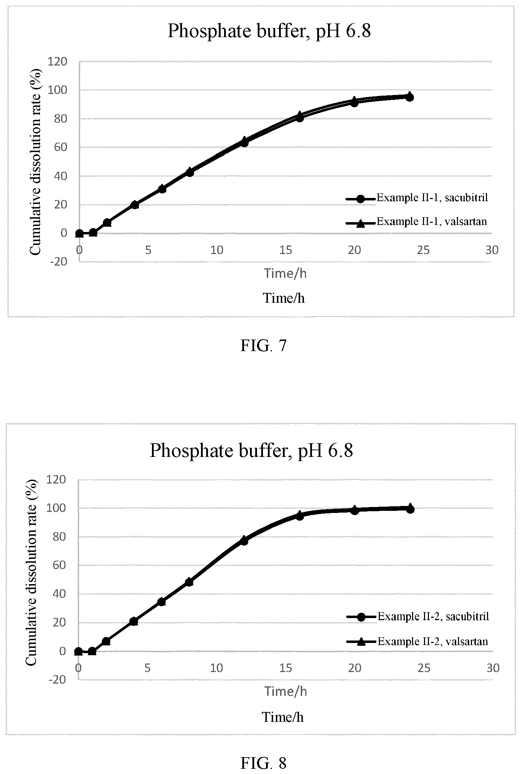

The dissolution data of the sacubitril valsartan sodium sustained-release tablets prepared by the above steps in a phosphate buffer at pH 6.8 are shown in Table 4 and FIG. 7.

**Table 4. Dissolution rates of sacubitril valsartan sodium sustained-release tablets prepared in Example II-1**

| Serial number | Example II-1 | |
|---|---|---|
| Active ingredient | Sacubitril | Valsartan |
| Time (h) | Cumulative dissolution rate (%) | |
| 1 | 0.8 | 0.8 |
| 2 | 7.5 | 7.7 |
| 4 | 19.8 | 20.3 |
| 6 | 30.8 | 31.6 |
| 8 | 42.5 | 43.7 |
| 12 | 63.1 | 65.0 |
| 16 | 80.5 | 82.7 |
| 20 | 91.0 | 93.1 |
| 24 | 95.1 | 96.5 |

### Example II-2

| Serial number | Example II-2 | |
|---|---|---|
| Name | Amount per tablet (mg) | Proportion (%) |
| **Drug-containing layer** | | |
| Sacubitril valsartan sodium | 113.10 | 40.39 |
| Colloidal silica | 2.00 | 0.71 |
| Copovidone | 75.30 | 26.90 |
| Hydroxyethylcellulose 250 L | 75.6 | 27.00 |
| Talc | 8.00 | 2.86 |
| Magnesium stearate | 6.00 | 2.14 |
| Total | 280.00 | 100.00 |

| **Boosting layer** | | |
|---|---|---|
| Polyoxyethylene | 73.00 | 60.83 |
| Copovidone | 10.00 | 8.33 |
| Iron oxide red | 1.00 | 0.83 |
| Sodium chloride | 35.00 | 29.17 |
| Magnesium stearate | 1.00 | 0.84 |
| Total | 120.00 | 100.00 |
| Weight of core | 400.00 | / |

| **Coating** | | |
|---|---|---|
| Cellulose acetate | 36.00 | 9.00 |
| Polyethylene glycol 4000 | 4.00 | 1.00 |
| Acetone | 684.00 | / |
| Purified water | 76.00 | / |

### Preparation procedure:

Sacubitril valsartan sodium described above was sieved with a 60-mesh sieve.

Step 1: The starting materials other than lubricant magnesium stearate in the drug-containing layer of the formula described above were added into a mixing tank and mixed at a mixing speed of 18 r/min for 20 min before the mixture was sized by dry granulation and magnesium stearate in the drug-containing layer was added; the mixture was mixed at a mixing speed of 18 r/min for 5 min to give a drug-containing layer mixture.

Step 2: Polyoxyethylene, sodium chloride, copovidone, and iron oxide red in the boosting layer of the formula described above were added into a mixing tank and mixed at a mixing speed of 18 r/min for 10 min before magnesium stearate in the boosting layer was added; the mixture was mixed at a mixing speed of 18 r/min for 5 min to give a boosting layer mixture.

Step 3: The drug-containing layer mixture obtained in Step 1 and the boosting layer mixture obtained in Step 2 were loaded on a rotary tablet press. The parameters of the press were adjusted such that the weight of the drug-containing layer was 280 mg, the weight of the boosting layer was 120 mg, the total weight of the tablet was 400 mg, and the rigidity was 100 N to 180 N. The mixtures were tabletted into shallow-convex round drug-containing cores.

### Step 4:

A sustained-release coating solution was prepared according to the following steps: Purified water was added to a beaker and stirred by a magnetic stirrer with acetone added during the stirring. The mixture was uniformly stirred for another 5 minutes after the addition of acetone was completed. Opadry^{®} CA was added slowly to the mixture and the mixture was continuously stirred for 30 to 90 minutes for a complete dissolution to give the sustained-release coating solution.

The drug-containing cores obtained in Step 3 were coated with the sustained-release coating solution described above. At a rotation speed of 8 rpm to 15 rpm, a tablet bed temperature of 25 °C to 35 °C, and a spraying speed of 5 rpm to 20 rpm, the solution was sprayed for coating until the coating weight gain was 8.0% to 11.0%. The tablet cores were dried at a tablet bed temperature of 30 °C to 35 °C for 5 to 10 minutes, dried in an air blast dryer at 45 °C, and aged for 24 hours to give the coated tablets.

Step 5: On the drug-containing layer (white) of the coated tablets obtained in Step 4, a pore was formed by laser with a size of 0.4 to 0.7 mm to give the sacubitril valsartan sodium sustained-release tablets.

The dissolution data of the sacubitril valsartan sodium sustained-release tablets prepared by the above steps in a phosphate buffer at pH 6.8 are shown in Table 5 and FIG. 8.

**Table 5. Dissolution rates of sacubitril valsartan sodium sustained-release tablets prepared in Example II-2**

| Serial number | Example II-2 | |
|---|---|---|
| Active ingredient | Sacubitril | Valsartan |
| Time (h) | Cumulative dissolution rate (%) | |
| 1 | 0.3 | 0.3 |
| 2 | 7.1 | 7.2 |
| 4 | 20.9 | 21.1 |
| 6 | 34.4 | 34.9 |
| 8 | 48.0 | 48.8 |
| 12 | 76.9 | 78.4 |
| 16 | 94.3 | 95.7 |
| 20 | 98.3 | 99.4 |
| 24 | 99.3 | 100.8 |

### Example II-3

| Serial number | Example II-3 | |
|---|---|---|
| Name | Amount per tablet (mg) | Proportion (%) |
| **Drug-containing layer** | | |
| Sacubitril valsartan sodium | 113.10 | 37.70 |
| Colloidal silica | 2.00 | 0.67 |
| Copovidone | 84.60 | 28.20 |
| Hydroxyethylcellulose | 86.00 | 28.66 |
| Talc | 8.00 | 2.67 |
| Iron oxide yellow | 0.30 | 0.10 |
| Magnesium stearate | 6.00 | 2.00 |
| Total | 300.00 | 100.00 |

| **Boosting layer** | | |
|---|---|---|
| Polyoxyethylene | 63.00 | 63.00 |
| Copovidone | 10.00 | 10.00 |
| Iron oxide red | 1.00 | 1.00 |
| Sodium chloride | 25.00 | 25.00 |
| Magnesium stearate | 1.00 | 1.00 |
| Total | 100.00 | 100.00 |
| Weight of core | 400.00 | / |

| **Coating** | | |
|---|---|---|
| Cellulose acetate | 36.00 | 9.00 |
| Polyethylene glycol 4000 | 4.00 | 1.00 |
| Acetone | 684.00 | / |
| Purified water | 76.00 | / |

### Preparation procedure:

Sacubitril valsartan sodium described above was sieved with a 60-mesh sieve.

Step 1: sacubitril valsartan sodium, copovidone, hydroxyethylcellulose, iron oxide yellow, colloidal silica, and talc in the drug-containing layer of the formula described above were added into a mixing tank and mixed at a mixing speed of 18 r/min for 20 min before the mixture was sized by dry granulation and magnesium stearate in the drug-containing layer was added; the mixture was mixed at a mixing speed of 18 r/min for 5 min to give a drug-containing layer mixture.

Step 2: Polyoxyethylene, sodium chloride, copovidone, and iron oxide red in the boosting layer of the formula described above were added into a mixing tank and mixed at a mixing speed of 18 r/min for 10 min before magnesium stearate in the boosting layer was added; the mixture was mixed at a mixing speed of 18 r/min for 5 min to give a boosting layer mixture.

Step 3: The drug-containing layer mixture obtained in Step 1 and the boosting layer mixture obtained in Step 2 were loaded on a rotary tablet press. The parameters of the press were adjusted such that the weight of the drug-containing layer was 300 mg, the weight of the boosting layer was 100 mg, the total weight of the tablet was 400 mg, and the rigidity was 100 N to 180 N. The mixtures were tabletted into shallow-convex round drug-containing cores.

### Step 4:

A sustained-release coating solution was prepared according to the following steps: Purified water was added to a beaker and stirred by a magnetic stirrer with acetone added during the stirring. The mixture was uniformly stirred for another 5 minutes after the addition of acetone was completed. Opadry^{®} CA was added slowly to the mixture and the mixture was continuously stirred for 30 to 90 minutes for a complete dissolution to give the sustained-release coating solution.

The drug-containing cores obtained in Step 3 were coated with the sustained-release coating solution described above. At a rotation speed of 8 rpm to 15 rpm, a tablet bed temperature of 25 °C to 35 °C, and a spraying speed of 5 rpm to 20 rpm, the solution was sprayed for coating until the coating weight gain was 8.0% to 14.0%. The tablet cores were dried at a tablet bed temperature of 30 °C to 35 °C for 5 to 10 minutes, dried in an air blast dryer at 45 °C, and aged for 24 hours to give the coated tablets.

Step 5: On the drug-containing layer (white) of the coated tablets obtained in Step 4, a pore was formed by laser with a size of 0.4 to 0.7 mm to give the sacubitril valsartan sodium sustained-release tablets.

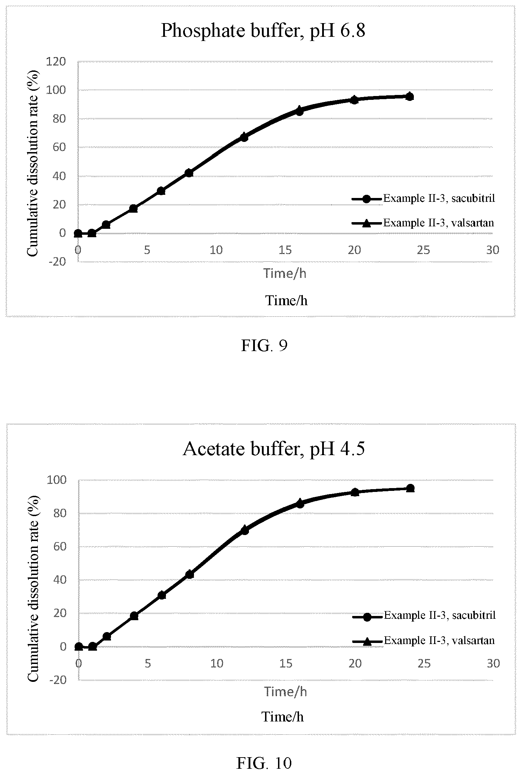

The dissolution data of the sacubitril valsartan sodium sustained-release tablets prepared by the above steps in a phosphate buffer at pH 6.8, water, and an acetate medium at pH 4.5 are shown in Table 6 and FIGs. 9, 11 and 10.

**Table 6. Dissolution rates of sacubitril valsartan sodium sustained-release tablets prepared in Example II-3**

| Serial number | Example II-3 | | | | | |
|---|---|---|---|---|---|---|
| Medium | Phosphate, pH 6.8 | | Water | | Acetate, pH 4.5 | |
| Active ingredient | Sacubitril | Valsartan | Sacubitril | Valsartan | Sacubitril | Valsartan |
| Time (h) | Cumulative dissolution rate (%) | | Cumulative dissolution rate (%) | | Cumulative dissolution rate (%) | |
| 1 | 0.3 | 0.3 | 0.3 | 0.3 | 0.2 | 0.1 |
| 2 | 6.1 | 6.1 | 6.5 | 6.3 | 6.2 | 6.1 |
| 4 | 17.4 | 17.5 | 18.8 | 18.7 | 18.4 | 18.4 |
| 6 | 29.6 | 30.0 | 32.3 | 32.5 | 30.6 | 31 |
| 8 | 42.1 | 42.8 | 45.3 | 46.0 | 42.9 | 43.6 |
| 12 | 66.7 | 68.1 | 71.0 | 72.2 | 69.3 | 70.5 |
| 16 | 84.9 | 86.6 | 87.6 | 88.9 | 85.4 | 86.6 |
| 20 | 93.0 | 93.9 | 93.8 | 94.1 | 92.4 | 92.8 |
| 24 | 95.6 | 96.2 | 95.7 | 96.2 | 95.0 | 95.1 |

### Example II-4

| Serial number | Example II-4 | |
|---|---|---|
| Name | Amount per tablet (mg) | Proportion (%) |
| **Drug-containing layer** | | |
| Sacubitril valsartan sodium | 226.20 | 75.40 |
| Colloidal silica | 2.00 | 0.67 |
| Copovidone | 34.80 | 11.60 |
| Hydroxyethylcellulose | 25.00 | 8.33 |
| Talc | 3.00 | 1.00 |
| Magnesium stearate | 9.00 | 3.00 |
| Total | 300.00 | 100.00 |

| **Boosting layer** | | |
|---|---|---|
| Polyoxyethylene | 73.00 | 73.00 |
| Copovidone | 20.00 | 20.00 |
| Iron oxide red | 1.00 | 1.00 |
| Sodium chloride | 5.00 | 5.00 |
| Magnesium stearate | 1.00 | 1.00 |
| Total | 100.00 | 100.00 |
| Weight of core | 400.00 | / |

| **Coating** | | |
|---|---|---|
| Cellulose acetate | 36.00 | 9.00 |
| Polyethylene glycol 4000 | 4.00 | 1.00 |
| Acetone | 684.00 | / |
| Purified water | 76.00 | / |

### Preparation procedure:

Sacubitril valsartan sodium described above was sieved with a 60-mesh sieve.

Step 1: sacubitril valsartan sodium, copovidone, hydroxyethylcellulose, colloidal silica, and talc in the drug-containing layer of the formula described above were added into a mixing tank and mixed at a mixing speed of 18 r/min for 20 min before the mixture was sized by dry granulation and magnesium stearate in the drug-containing layer was added; the mixture was mixed at a mixing speed of 18 r/min for 5 min to give a drug-containing layer mixture.

Step 2: Polyoxyethylene, sodium chloride, copovidone, and iron oxide red in the boosting layer of the formula described above were added into a mixing tank and mixed at a mixing speed of 18 r/min for 10 min before magnesium stearate in the boosting layer was added; the mixture was mixed at a mixing speed of 18 r/min for 5 min to give a boosting layer mixture.

Step 3: The drug-containing layer mixture obtained in Step 1 and the boosting layer mixture obtained in Step 2 were loaded on a rotary tablet press. The parameters of the press were adjusted such that the weight of the drug-containing layer was 300 mg, the weight of the boosting layer was 100 mg, the total weight of the tablet was 400 mg, and the rigidity was 100 N to 180 N. The mixtures were tabletted into shallow-convex round drug-containing cores.

### Step 4:

A sustained-release coating solution was prepared according to the following steps: Purified water was added to a beaker and stirred by a magnetic stirrer with acetone added during the stirring. The mixture was uniformly stirred for another 5 minutes after the addition of acetone was completed. Opadry^{®} CA was added slowly to the mixture and the mixture was continuously stirred for 30 to 90 minutes for a complete dissolution to give the sustained-release coating solution.

The drug-containing cores obtained in Step 3 were coated with the sustained-release coating solution described above. At a rotation speed of 8 rpm to 15 rpm, a tablet bed temperature of 25 °C to 35 °C, and a spraying speed of 5 rpm to 20 rpm, the solution was sprayed for coating until the coating weight gain was 8.0% to 11.0%. The tablet cores were dried at a tablet bed temperature of 30 °C to 35 °C for 5 to 10 minutes, dried in an air blast dryer at 45 °C, and aged for 24 hours to give the coated tablets.

Step 5: On the drug-containing layer (white) of the coated tablets obtained in Step 4, a pore was formed by laser with a size of 0.4 to 0.7 mm to give the sacubitril valsartan sodium sustained-release tablets.

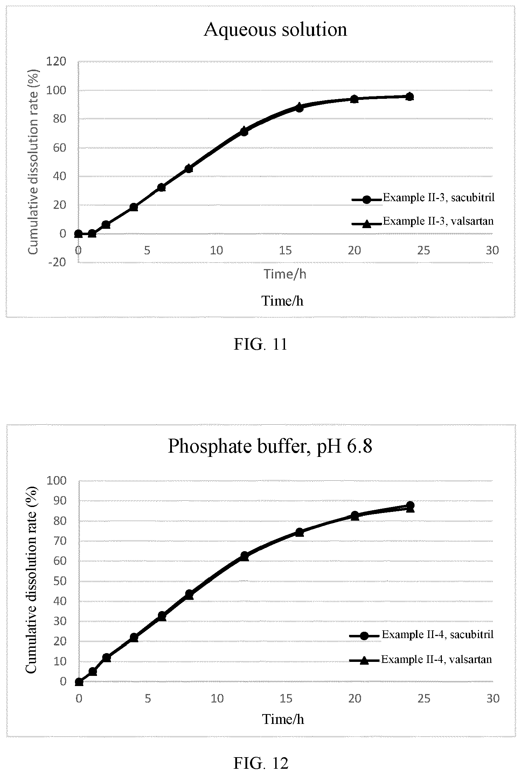

The dissolution data of the sacubitril valsartan sodium sustained-release tablets prepared by the above steps in a phosphate buffer at pH 6.8 is shown in Table 7 and FIG. 12.

**Table 7. Dissolution rates of sacubitril valsartan sodium sustained-release tablets prepared in Example II-4**

| Serial number | Example II-4 | |
|---|---|---|
| Active ingredient | Sacubitril | Valsartan |
| Time (h) | Cumulative dissolution rate (%) | |
| 1 | 5.1 | 5 |
| 2 | 12.2 | 11.9 |
| 4 | 22.2 | 21.6 |
| 6 | 33 | 32.1 |
| 8 | 43.9 | 42.9 |
| 12 | 62.9 | 62.0 |
| 16 | 74.7 | 74.3 |
| 20 | 82.8 | 82.3 |
| 24 | 87.8 | 86.2 |

### Example II-5

| Serial number | Example II-5 | |
|---|---|---|
| Name | Amount per tablet (mg) | Proportion (%) |
| **Drug-containing layer** | | |
| Sacubitril valsartan sodium | 226.2 | 75.40 |
| Colloidal silica | 2 | 0.67 |
| Polyoxyethylene | 59.8 | 19.93 |
| Talc | 3.00 | 1.00 |
| Magnesium stearate | 9.00 | 3.00 |
| Total | 300.00 | 100.00 |

| **Boosting layer** | | |
|---|---|---|
| Polyoxyethylene | 82.00 | 82.00 |
| Copovidone | 10.00 | 10.00 |
| Iron oxide red | 1.00 | 1.00 |
| Sodium chloride | 5.00 | 5.00 |
| Magnesium stearate | 2.00 | 2.00 |
| Total | 100.00 | 100.00 |
| Weight of core | 400.00 | / |

| **Coating** | | |
|---|---|---|
| Cellulose acetate | 36.00 | 9.00 |
| Polyethylene glycol 4000 | 4.00 | 1.00 |
| Acetone | 684.00 | / |
| Purified water | 76.00 | / |

### Preparation procedure:

Sacubitril valsartan sodium described above was sieved with a 60-mesh sieve.

Step 1: The starting materials other than lubricant magnesium stearate in the drug-containing layer of the formula described above were added into a mixing tank and mixed at a mixing speed of 18 r/min for 20 min before the mixture was sized by dry granulation and magnesium stearate in the drug-containing layer was added; the mixture was mixed at a mixing speed of 18 r/min for 5 min to give a drug-containing layer mixture.

Step 2: Polyoxyethylene, sodium chloride, copovidone, and iron oxide red in the boosting layer of the formula described above were added into a mixing tank and mixed at a mixing speed of 18 r/min for 10 min before magnesium stearate in the boosting layer was added; the mixture was mixed at a mixing speed of 18 r/min for 5 min to give a boosting layer mixture.

Step 3: The drug-containing layer mixture obtained in Step 1 and the boosting layer mixture obtained in Step 2 were loaded on a rotary tablet press. The parameters of the press were adjusted for tabletting according to the target weights of the drug-containing layer and the boosting layer, so as to give 400-mg shallow-convex round drug-containing cores with a rigidity of 100 N to 180N.

### Step 4:

A sustained-release coating solution was prepared according to the following steps: Purified water was added to a beaker and stirred by a magnetic stirrer with acetone added during the stirring. The mixture was uniformly stirred for another 5 minutes after the addition of acetone was completed. Opadry^{®} CA was added slowly to the mixture and the mixture was continuously stirred for 30 to 90 minutes for a complete dissolution to give the sustained-release coating solution.

The drug-containing cores obtained in Step 3 were coated with the sustained-release coating solution described above. At a rotation speed of 8 rpm to 15 rpm, a tablet bed temperature of 25 °C to 35 °C, and a spraying speed of 5 rpm to 20 rpm, the solution was sprayed for coating until the coating weight gain was 8.0% to 11.0%. The tablet cores were dried at a tablet bed temperature of 30 °C to 35 °C for 5 to 10 minutes, dried in an air blast dryer at 45 °C, and aged for 24 hours to give the coated tablets.

Step 5: On the drug-containing layer (white) of the coated tablets obtained in Step 4, a pore was formed by laser with a size of 0.4 to 0.7 mm to give the sacubitril valsartan sodium sustained-release tablets.

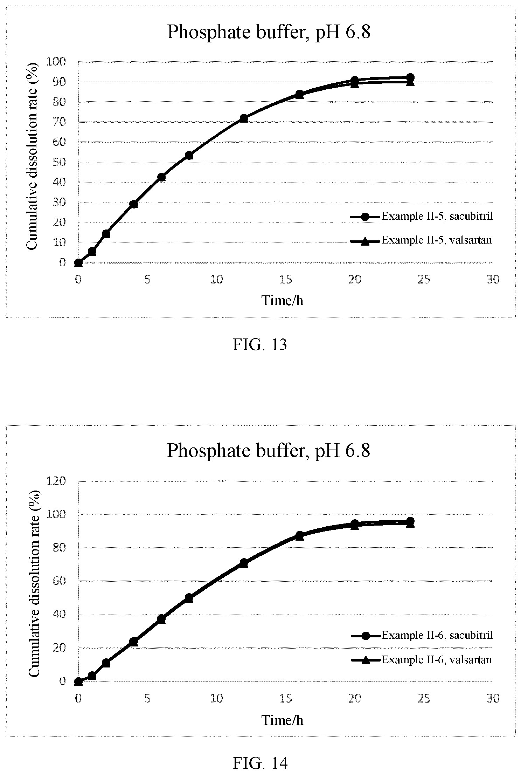

The dissolution data of the sacubitril valsartan sodium sustained-release tablets produced by the above steps in a phosphate buffer at pH 6.8 is shown in Table 8 and FIG. 13.

**Table 8. Dissolution rates of sacubitril valsartan sodium sustained-release tablets prepared in Example II-5**

| Serial number | Example II-5 | |
|---|---|---|
| Active ingredient | Sacubitril | Valsartan |
| Time (h) | Cumulative dissolution rate (%) | |
| 1 | 5.7 | 5.6 |
| 2 | 14.3 | 14.4 |
| 4 | 29.1 | 29.3 |
| 6 | 42.6 | 42.8 |
| 8 | 53.5 | 53.6 |
| 12 | 72.0 | 71.9 |
| 16 | 84.2 | 83.6 |
| 20 | 90.9 | 89.2 |
| 24 | 92.3 | 90.1 |

### Example II-6

| Serial number | Example II-6 | |
|---|---|---|
| Name | Amount per tablet (mg) | Proportion (%) |
| **Drug-containing layer** | | |
| Sacubitril valsartan sodium | 226.20 | 75.40 |
| Colloidal silica | 2.00 | 0.67 |
| Copovidone | 20.50 | 6.83 |
| Hydroxyethylcellulose | 36.00 | 12.00 |
| Talc | 8.00 | 2.67 |
| Iron oxide yellow | 0.30 | 0.10 |
| Magnesium stearate | 7.00 | 2.33 |
| Total | 300.00 | 100.00 |

| **Boosting layer** | | |
|---|---|---|
| Polyoxyethylene | 63.00 | 63.00 |
| Copovidone | 10.00 | 10.00 |
| Iron oxide red | 1.00 | 1.00 |
| Sodium chloride | 25.00 | 25.00 |
| Magnesium stearate | 1.00 | 1.00 |
| Total | 100.00 | 100.00 |
| Weight of core | 400.00 | / |

| **Coating** | | |
|---|---|---|
| Cellulose acetate | 36.00 | 9.00 |
| Polyethylene glycol 4000 | 4.00 | 1.00 |
| Acetone | 684.00 | / |
| Purified water | 76.00 | / |

### Preparation procedure:

Sacubitril valsartan sodium described above was sieved with a 60-mesh sieve.

Step 1: sacubitril valsartan sodium, copovidone, hydroxyethylcellulose, iron oxide yellow, colloidal silica, and talc in the drug-containing layer of the formula described above were added into a mixing tank and mixed at a mixing speed of 18 r/min for 20 min before the mixture was sized by dry granulation and magnesium stearate in the drug-containing layer was added; the mixture was mixed at a mixing speed of 18 r/min for 5 min to give a drug-containing layer mixture.

Step 2: Polyoxyethylene, sodium chloride, copovidone, and iron oxide red in the boosting layer of the formula described above were added into a mixing tank and mixed at a mixing speed of 18 r/min for 10 min before magnesium stearate in the boosting layer was added; the mixture was mixed at a mixing speed of 18 r/min for 5 min to give a boosting layer mixture.

Step 3: The drug-containing layer mixture obtained in Step 1 and the boosting layer mixture obtained in Step 2 were loaded on a rotary tablet press. The parameters of the press were adjusted such that the weight of the drug-containing layer was 300 mg, the weight of the boosting layer was 100 mg, the total weight of the tablet was 400 mg, and the rigidity was 100 N to 180 N. The mixtures were tabletted into shallow-convex round drug-containing cores.

### Step 4:

A sustained-release coating solution was prepared according to the following steps: Purified water was added to a beaker and stirred by a magnetic stirrer with acetone added during the stirring. The mixture was uniformly stirred for another 5 minutes after the addition of acetone was completed. Opadry^{®} CA was added slowly to the mixture and the mixture was continuously stirred for 30 to 90 minutes for a complete dissolution to give the sustained-release coating solution.

The drug-containing cores obtained in Step 3 were coated with the sustained-release coating solution described above. At a rotation speed of 8 rpm to 15 rpm, a tablet bed temperature of 25 °C to 35 °C, and a spraying speed of 5 rpm to 20 rpm, the solution was sprayed for coating until the coating weight gain was 8.0% to 14.0%. The tablet cores were dried at a tablet bed temperature of 30 °C to 35 °C for 5 to 10 minutes, dried in an air blast dryer at 45 °C, and aged for 24 hours to give the coated tablets.

Step 5: On the drug-containing layer (white) of the coated tablets obtained in Step 4, a pore was formed by laser with a size of 0.4 to 0.7 mm to give the sacubitril valsartan sodium sustained-release tablets.

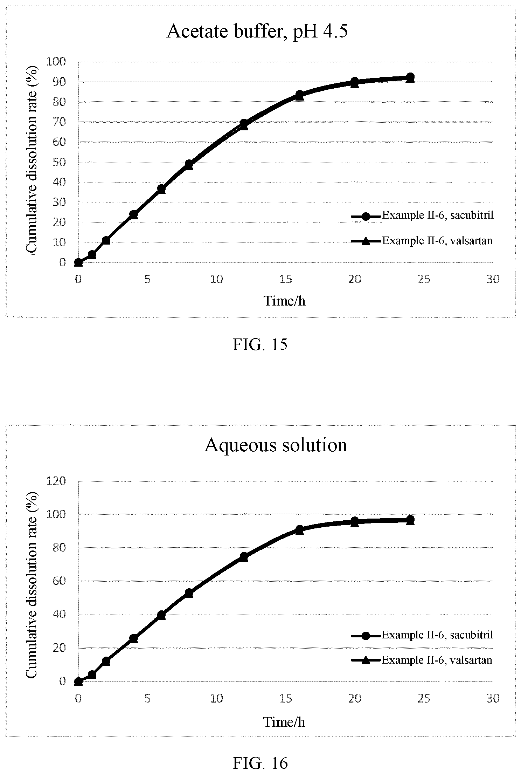

The dissolution data of the sacubitril valsartan sodium sustained-release tablets prepared by the above steps in a phosphate buffer at pH 6.8, water, and an acetate medium at pH 4.5 are shown in Table 9 and FIGs. 14, 16 and 15. This indicates that the sustained-release tablets release the active ingredients in normal physiological conditions without being affected by the medium.

**Table 9. Dissolution rates of sacubitril valsartan sodium sustained-release tablets prepared in Example II-6**

| Serial number | Example II-6 | | | | | |
|---|---|---|---|---|---|---|
| Medium | Phosphate, pH 6.8 | | Water | | Acetate, pH 4.5 | |
| Active ingredient | Sacubitril | Valsartan | Sacubitril | Valsartan | Sacubitril | Valsartan |
| Time (h) | Cumulative dissolution rate (%) | | Cumulative dissolution rate (%) | | Cumulative dissolution rate (%) | |
| 1 | 3.6 | 3.5 | 4.2 | 4.1 | 4.1 | 3.9 |
| 2 | 11.2 | 10.9 | 12.3 | 12.0 | 11.1 | 11.1 |
| 4 | 24.1 | 23.5 | 26.1 | 25.5 | 24.1 | 23.5 |
| 6 | 37.7 | 36.9 | 40.0 | 39.3 | 36.9 | 36.1 |
| 8 | 50.3 | 49.4 | 53.2 | 52.4 | 49.4 | 48.1 |
| 12 | 71.4 | 70.5 | 75.1 | 74.1 | 69.6 | 68.0 |
| 16 | 87.6 | 86.7 | 91.1 | 90.1 | 83.9 | 82.8 |
| 20 | 94.6 | 93.0 | 96.1 | 94.8 | 90.4 | 89.2 |
| 24 | 96.0 | 94.5 | 97.1 | 95.9 | 92.5 | 91.8 |

### Example II-7

Sacubitril valsartan sodium was prepared into gastroretentive sustained-release tablets. The formula is designed as follows:

| Serial number | Example II-7 | |
|---|---|---|
| Name | Amount per tablet (mg) | Proportion (%) |
| Sacubitril valsartan sodium | 226.20 | 22.62 |
| Polyvinyl acetate povidone mixture | 320.00 | 32.00 |
| Crospovidone | 80.00 | 8.00 |
| Polyoxyethylene | 150.00 | 15.00 |
| Hydroxypropyl methylcellulose K4M | 60.00 | 6.00 |
| Lactose monohydrate | 153.80 | 15.38 |
| Magnesium stearate | 10.00 | 1.00 |
| Total | 1000 | 100.00 |

### Preparation procedure:

Sacubitril valsartan sodium described above was sieved with a 60-mesh sieve.

Step 1: The starting materials other than the lubricant in the formula described above were added into a mixing tank and mixed at a mixing speed of 18 r/min for 20 min before magnesium stearate was added according to the formula; the mixture was mixed at a mixing speed of 18 r/min for 5 min to give a material mixture.

Step 2: The mixture obtained in Step 1 was loaded on a rotary tablet press. The parameters of the press were adjusted for tabletting according to a tablet weight of 1000 mg and a rigidity of 140 N to 220N.

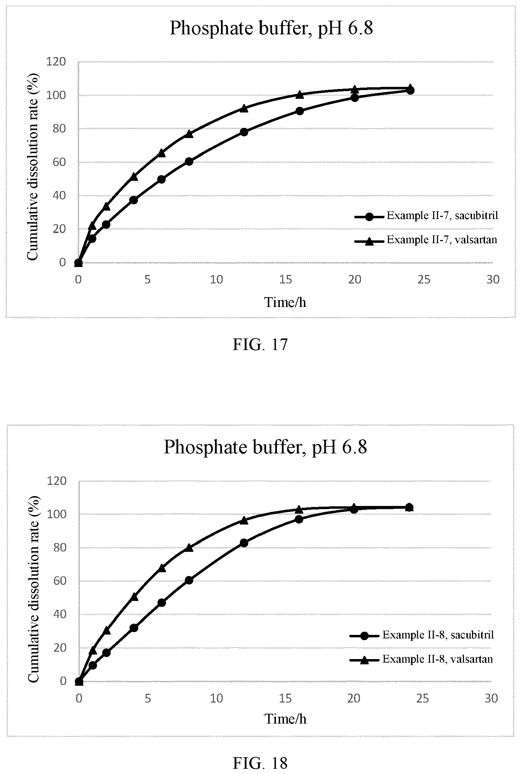

The dissolution data of the sacubitril valsartan sodium sustained-release tablets produced by the above steps in a phosphate buffer at pH 6.8 is shown in Table 10 and FIG. 17.

**Table 10. Dissolution rates of sacubitril valsartan sodium sustained-release tablets prepared in Example II-7**

| Serial number | Example II-7 | |
|---|---|---|
| Active ingredient | Sacubitril | Valsartan |
| Time (h) | Cumulative dissolution rate (%) | |
| 1 | 14.4 | 22.0 |
| 2 | 22.8 | 33.6 |
| 4 | 37.4 | 51.6 |
| 6 | 49.8 | 65.6 |
| 8 | 60.6 | 76.9 |
| 12 | 78.1 | 92.3 |
| 16 | 90.7 | 100.6 |
| 20 | 98.6 | 103.8 |
| 24 | 103.1 | 104.6 |

### Example II-8

Sacubitril valsartan sodium was prepared into common gel-matrix sustained-release tablets. The formula is designed as follows:

| Serial number | Example II-8 | |
|---|---|---|
| Name | Amount per tablet (mg) | Proportion (%) |
| Sacubitril valsartan sodium | 226.20 | 45.24 |
| Hydroxypropyl methylcellulose 100LV | 50.00 | 10.00 |
| Hydroxypropyl methylcellulose K4M | 150.00 | 30.00 |
| Lactose monohydrate | 68.80 | 13.76 |
| Magnesium stearate | 5.00 | 1.00 |
| Total | 500.00 | 100.00 |

### Preparation procedure:

Sacubitril valsartan sodium described above was sieved with a 60-mesh sieve.

Step 1: The starting materials other than the lubricant in the formula described above were added into a mixing tank and mixed at a mixing speed of 18 r/min for 20 min before magnesium stearate was added according to the formula; the mixture was mixed at a mixing speed of 18 r/min for 5 min to give a material mixture.

Step 2: The mixture obtained in Step 1 was loaded on a rotary tablet press. The parameters of the press were adjusted for tabletting according to a tablet weight of 500mg and a rigidity of 100 N to 180N.

The dissolution data of the sacubitril valsartan sodium sustained-release tablets produced by the above steps in a phosphate buffer at pH 6.8 is shown in Table 11 and FIG. 18.

**Table 11. Dissolution rates of sacubitril valsartan sodium sustained-release tablets prepared in Example II-8**

| Serial number | Example II-8 | |
|---|---|---|
| Active ingredient | Sacubitril | Valsartan |
| Time (h) | Cumulative dissolution rate (%) | |
| 1 | 9.7 | 18.6 |
| 2 | 17.3 | 30.6 |
| 4 | 32.1 | 50.7 |
| 6 | 47.1 | 68.0 |
| 8 | 60.5 | 80.1 |
| 12 | 82.9 | 96.5 |
| 16 | 97.1 | 103 |
| 20 | 103.0 | 104.2 |
| 24 | 104.2 | 104.5 |

As can be seen from the results of Examples II-7 and II-8, sacubitril and valsartan greatly differ in the release rate and cannot be released synchronously from gastroretentive sustained-release tablets and common gel-matrix sustained-release tablets in *in vitro* dissolution conditions.

As can be seen from the results of Examples I-1 to I-6 and Examples II-1 to II-6, sacubitril and valsartan in the sacubitril valsartan sodium sustained-release tablets of the osmotic pump form of the present disclosure can be released synchronously with a long-acting effect in *in vitro* dissolution conditions, which can meet the requirement of a q.d. regimen; the drug release performance is not influenced by the pH of the medium.

The above examples illustrate the embodiments of the present disclosure. However, the present disclosure is not limited to the embodiments described above. Any modification, equivalent, improvement, and the like made without departing from the spirit and principle of the present disclosure shall fall within the protection scope of the present disclosure.

## Claims

1. A sacubitril valsartan sodium sustained-release composition, wherein the sustained-release composition is a 24-hour sustained-release drug, and the dissolution of sacubitril and valsartan simultaneously meets the following three features:
A) no more than 40% of the active pharmaceutical ingredient is dissolved within 1 hour;
B) 10% to 70% of the active pharmaceutical ingredient is dissolved within 6 hours; and
C) no less than 65% of the active pharmaceutical ingredient is dissolved within 24 hours;
the active pharmaceutical ingredients can be selected from one, two, or more of sacubitril valsartan sodium, or other pharmaceutically acceptable salts, solvates, and hydrates thereof;
preferably, in the sacubitril valsartan sodium sustained-release composition, the dissolution of sacubitril simultaneously meets the following three features:
A) no more than 35% of the active pharmaceutical ingredient is dissolved within 1 hour;
B) 10% to 70% of the active pharmaceutical ingredient is dissolved within 6 hours; and
C) no less than 70% of the active pharmaceutical ingredient is dissolved within 24 hours;
the dissolution of valsartan simultaneously meets the following three features:
A) no more than 35% of the active pharmaceutical ingredient is dissolved within 1 hour;
B) 15% to 70% of the active pharmaceutical ingredient is dissolved within 6 hours; and
C) no less than 75% of the active pharmaceutical ingredient is dissolved within 24 hours;
preferably, in the sacubitril valsartan sodium sustained-release composition, the dissolution of sacubitril simultaneously meets the following three features:
A) no more than 25% of the active pharmaceutical ingredient is dissolved within 1 hour;
B) 10% to 65% of the active pharmaceutical ingredient is dissolved within 6 hours; and
C) no less than 75% of the active pharmaceutical ingredient is dissolved within 24 hours;
the dissolution of valsartan simultaneously meets the following three features:
A) no more than 30% of the active pharmaceutical ingredient is dissolved within 1 hour;
B) 10% to 65% of the active pharmaceutical ingredient is dissolved within 6 hours; and
C) no less than 75% of the active pharmaceutical ingredient is dissolved within 24 hours;
preferably, in the sacubitril valsartan sodium sustained-release composition, the dissolution of sacubitril simultaneously meets the following three features:
A) no more than 25% of the active pharmaceutical ingredient is dissolved within 1 hour;
B) 10% to 65% of the active pharmaceutical ingredient is dissolved within 6 hours; and
C) no less than 80% of the active pharmaceutical ingredient is dissolved within 24 hours;
the dissolution of valsartan simultaneously meets the following three features:
A) no more than 25% of the active pharmaceutical ingredient is dissolved within 1 hour;
B) 15% to 65% of the active pharmaceutical ingredient is dissolved within 6 hours; and
C) no less than 80% of the active pharmaceutical ingredient is dissolved within 24 hours;
preferably, the dissolution of sacubitril and valsartan is a synchronic release.

2. A sacubitril valsartan sodium sustained-release composition, wherein the sustained-release composition is a 24-hour sustained-release drug, and the dissolution of sacubitril and valsartan simultaneously meets the following three features:
A) no more than 40% of the active pharmaceutical ingredient is dissolved within 2 hours;
B) 20% to 75% of the active pharmaceutical ingredient is dissolved within 8 hours; and
C) no less than 65% of the active pharmaceutical ingredient is dissolved within 24 hours;
the active pharmaceutical ingredients can be selected from one, two, or more of sacubitril valsartan sodium, or other pharmaceutically acceptable salts, solvates, and hydrates thereof;
preferably, in the sacubitril valsartan sodium sustained-release composition, the dissolution of sacubitril simultaneously meets the following three features:
A) no more than 35% of the active pharmaceutical ingredient is dissolved within 2 hours;
B) 20% to 70% of the active pharmaceutical ingredient is dissolved within 8 hours; and
C) no less than 75% of the active pharmaceutical ingredient is dissolved within 24 hours;
the dissolution of valsartan simultaneously meets the following three features:
A) no more than 35% of the active pharmaceutical ingredient is dissolved within 2 hours;
B) 20% to 70% of the active pharmaceutical ingredient is dissolved within 8 hours; and
C) no less than 75% of the active pharmaceutical ingredient is dissolved within 24 hours;
preferably, in the sacubitril valsartan sodium sustained-release composition, the dissolution of sacubitril simultaneously meets the following three features:
A) no more than 25% of the active pharmaceutical ingredient is dissolved within 2 hours;
B) 25% to 65% of the active pharmaceutical ingredient is dissolved within 8 hours; and
C) no less than 75% of the active pharmaceutical ingredient is dissolved within 24 hours;
the dissolution of valsartan simultaneously meets the following three features:
A) no more than 25% of the active pharmaceutical ingredient is dissolved within 2 hours;
B) 25% to 65% of the active pharmaceutical ingredient is dissolved within 8 hours; and
C) no less than 75% of the active pharmaceutical ingredient is dissolved within 24 hours;
preferably, in the sacubitril valsartan sodium sustained-release composition, the dissolution of sacubitril simultaneously meets the following three features:
A) no more than 25% of the active pharmaceutical ingredient is dissolved within 2 hours;
B) 30% to 65% of the active pharmaceutical ingredient is dissolved within 8 hours; and
C) no less than 80% of the active pharmaceutical ingredient is dissolved within 24 hours;
the dissolution of valsartan simultaneously meets the following three features:
A) no more than 25% of the active pharmaceutical ingredient is dissolved within 2 hours;
B) 30% to 65% of the active pharmaceutical ingredient is dissolved within 8 hours; and
C) no less than 80% of the active pharmaceutical ingredient is dissolved within 24 hours;
preferably, the dissolution of sacubitril and valsartan is a synchronic release.

3. A sacubitril valsartan sodium sustained-release composition, comprising a drug-containing layer core and a coating film, wherein a pore is provided on one side of the drug-containing layer; the drug-containing layer core comprises an active pharmaceutical ingredient and a carrier; the active pharmaceutical ingredient is selected from one, two, or more of sacubitril valsartan sodium, or other pharmaceutically acceptable salts, solvates, and hydrates thereof; the carrier is one or more of a tonicity adjusting agent, a sweller, thickener, a glidant, and a lubricant;
the sustained-release composition optionally further comprises or does not comprise a boosting layer core, and the boosting layer core comprises one or more of a tonicity adjusting agent, a sweller, a colorant and a lubricant; the coating film is a semipermeable film;
preferably, the sacubitril valsartan sodium sustained-release composition is the composition according to claim 1 or 2;
preferably, the drug-containing layer core further comprises a colorant.

4. The sacubitril valsartan sodium sustained-release composition according to claim 3, wherein:
in the drug-containing layer core, the active pharmaceutical ingredient is sacubitril valsartan sodium;
and/or,
in the drug-containing layer core, the tonicity adjusting agent is one or more of sodium chloride, potassium chloride, mannitol, sorbitol, sodium sulfate, magnesium sulfate, glucose, fructose, sucrose, and lactose;
and/or,
in the drug-containing layer core, the thickener is one or more of hydroxypropyl methylcellulose, hydroxypropylcellulose, hydroxyethylcellulose, ethyl cellulose, copovidone, acacia, pregelatinized starch, and polyvinylpyrrolidone;
and/or,
in the drug-containing layer core, the sweller is one or more of polyoxyethylene, carbomer, sodium carboxymethyl starch, crospovidone, and sodium alginate;
and/or,
in the drug-containing layer core, the colorant is selected from one or more of iron oxide red, iron oxide yellow, and iron oxide black;
and/or,
in the drug-containing layer core, the glidant is one or more of talc, colloidal silicon dioxide, and colloidal silica;
and/or,
in the drug-containing layer core, the lubricant is one or more of a metal stearate salt, stearic acid, talc, a stearate ester, stearyl fumarate, and colloidal silicon dioxide;
and/or,
in the boosting layer core, the tonicity adjusting agent is one or more of sodium chloride, potassium chloride, mannitol, sorbitol, sodium sulfate, magnesium sulfate, glucose, fructose, sucrose, and lactose;
and/or,
in the boosting layer core, the sweller is one or more of polyvinylpyrrolidone, hydroxyethylcellulose, copovidone, hydroxypropyl methylcellulose, acacia, carbomer, sodium carboxymethyl starch, sodium alginate, and polyoxyethylene;
and/or,
in the boosting layer core, the colorant is one or more of iron oxide red, iron oxide yellow, and iron oxide black;
and/or,
in the boosting layer core, the lubricant is selected from one or more of a metal stearate salt, stearic acid, talc, a stearate ester, stearyl fumarate, and colloidal silicon dioxide;
preferably, the metal stearate salt is magnesium stearate and/or calcium stearate;
and/or,
the stearate ester is glyceryl stearate.

5. The sacubitril valsartan sodium sustained-release composition according to claim 3 or 4, wherein:
in the drug-containing layer core, the content of the active pharmaceutical ingredient is 10.00% to 80.00%, on the weight basis of the drug-containing layer core;
and/or,
in the drug-containing layer core, the content of the tonicity adjusting agent is 1.00% to 60.00%, on the weight basis of the drug-containing layer core;
and/or,
in the drug-containing layer core, the content of the thickener is 10.00% to 70.00%, on the weight basis of the drug-containing layer core;
and/or,
in the drug-containing layer core, the content of the sweller is 0 to 70.00%, on the weight basis of the drug-containing layer core;
and/or,
in the drug-containing layer core, the content of the glidant is 0 to 5.00%, e.g., 0 to 1.00%, on the weight basis of the drug-containing layer core;
and/or,
in the drug-containing layer core, the content of the lubricant is 0.50% to 60.00%, on the weight basis of the drug-containing layer core;
and/or,
in the drug-containing layer core, the content of the colorant is 0 to 5.00%, on the weight basis of the drug-containing layer core.

6. The sacubitril valsartan sodium sustained-release composition according to any one of claims 3 to 5, wherein:
in the boosting layer core, the content of the tonicity adjusting agent is 1.00% to 60.00%, on the weight basis of the boosting layer core;
and/or,
in the boosting layer core, the content of the sweller is 0 to 90.00%, e.g., 0 to 80.00%, on the weight basis of the boosting layer core;
and/or,
in the boosting layer core, the content of the colorant is 0.10% to 10.00%, on the weight basis of the boosting layer core;
and/or,
in the boosting layer core, the content of the lubricant is 0.10% to 10.00%, on the weight basis of the boosting layer core.

7. The sacubitril valsartan sodium sustained-release composition according to any one of claims 3 to 6, wherein: the semipermeable film comprises one or more of a film-forming material, a porogen, and a plasticizer; preferably, in the coating film, the film-forming material is one or more of cellulose acetate, ethylcellulose, cellulose acetate phthalate, polyvinyl acetate, acrylic resin, and methacrylic resin;
and/or,
in the coating film, the porogen is one or more of polyethylene glycol, hydroxypropylcellulose, povidone, and polyvinyl alcohol;
and/or,
in the coating film, the plasticizer is one or more of glyceryl triacetate, glyceryl citrate, a glyceride, dibutyl phthalate, and diethyl phthalate;
preferably, the coating film is a fully formulated cellulose acetate coating premix provided by Colorcon.

8. The sacubitril valsartan sodium sustained-release composition according to any one of claims 3 to 7, wherein: the drug-containing layer core is selected from any one of the following formulas:
formula I: 28.25% of sacubitril valsartan sodium, 25.00% of sorbitol, 21.25% of lactose monohydrate, 2.50% of sodium chloride, 21.50% of hydroxypropyl methylcellulose, 0.50% of colloidal silica, and 1.00% of magnesium stearate, on the weight basis of the drug-containing layer core;
formula II: 56.50% of sacubitril valsartan sodium, 20.00% of sorbitol, 10.00% of lactose monohydrate, 1.50% of sodium chloride, 10.50% of hydroxypropyl methylcellulose, 0.50% of colloidal silica, and 1.00% of magnesium stearate, on the weight basis of the drug-containing layer core;
formula III: 37.67% of sacubitril valsartan sodium, 36.66% of sorbitol, 3.67% of sodium chloride, 21.00% of hydroxypropyl methylcellulose, and 1.00% of magnesium stearate, on the weight basis of the drug-containing layer core;
formula IV: 56.50% of sacubitril valsartan sodium, 25.00% of sorbitol, 2.50% of sodium chloride, 15.00% of hydroxypropyl methylcellulose, and 1.00% of magnesium stearate, on the weight basis of the drug-containing layer core;
formula V: 33.93% of sacubitril valsartan sodium, 48.06% of polyoxyethylene, 3.00% of sodium chloride, 13.81% of hydroxypropyl methylcellulose, and 1.20% of magnesium stearate, on the weight basis of the drug-containing layer core;
formula VI: 67.87% of sacubitril valsartan sodium, 24.02% of polyoxyethylene, 1.5% of sodium chloride, 5.41% of hydroxypropyl methylcellulose, and 1.20% of magnesium stearate, on the weight basis of the drug-containing layer core;
formula VII: 37.70% of sacubitril valsartan sodium, 38.63% of copovidone, 18.67% of hydroxyethylcellulose, 2.33% of talc, 0.67% of colloidal silica, and 2.00% of magnesium stearate, on the weight basis of the drug-containing layer core;
formula VIII: 75.40% of sacubitril valsartan sodium, 11.60% of copovidone, 8.33% of hydroxyethylcellulose, 1.00% of talc, 0.67% of colloidal silica, and 3.00% of magnesium stearate, on the weight basis of the drug-containing layer core;
formula IX: 40.39% of sacubitril valsartan sodium, 26.90% of copovidone, 27.00% of hydroxyethylcellulose, 2.86% of talc, 0.71% of colloidal silica, and 2.14% of magnesium stearate, on the weight basis of the drug-containing layer core;
formula X: 75.40% of sacubitril valsartan sodium, 19.93% of polyoxyethylene, 1.00% of talc, 0.67% of colloidal silica, and 3.00% of magnesium stearate, on the weight basis of the drug-containing layer core;
formula XI: 37.70% of sacubitril valsartan sodium, 28.20% of copovidone, 28.66% of hydroxyethylcellulose, 2.67% of talc, 0.67% of colloidal silica, 2.00% of magnesium stearate, and 0.10% of iron oxide yellow, on the weight basis of the drug-containing layer core; and
formula XII: 75.40% of sacubitril valsartan sodium, 6.83% of copovidone, 12.00% of hydroxyethylcellulose, 2.67% of talc, 0.67% of colloidal silica, 2.33% of magnesium stearate, and 0.10% of iron oxide yellow, on the weight basis of the drug-containing layer core;
and/or, the boosting layer core is selected from any one of the following formulas:
formula 1: 65.86% of polyoxyethylene, 31.74% of sodium chloride, 1.2% of iron oxide red, and 1.20% of magnesium stearate, on the weight basis of the boosting layer core;
formula 2: 73.00% of polyoxyethylene, 20.00% of copovidone, 1.00% of iron oxide red, 5.00% of sodium chloride, and 1.00% of magnesium stearate, on the weight basis of the boosting layer core;
formula 3: 60.83% of polyoxyethylene, 8.33% of copovidone, 29.17% of sodium chloride, 0.83% of iron oxide red, and 0.84% of magnesium stearate, on the weight basis of the boosting layer core;
formula 4: 82.00% of polyoxyethylene, 10.00% of copovidone, 5.00% of sodium chloride, 1.00% of iron oxide red, and 2.00% of magnesium stearate, on the weight basis of the boosting layer core; and
formula 5: 63.00% of polyoxyethylene, 10.00% of copovidone, 25.00% of sodium chloride, 1.00% of iron oxide red, and 1.00% of magnesium stearate, on the weight basis of the boosting layer core;
and/or, the coating is any one of the following compositions:
coating I: 9.00% of cellulose acetate and 1.00% of polyethylene glycol 4000, on the weight basis of the tablet core;
coating II: 7.20% of cellulose acetate and 0.80% of polyethylene glycol 4000, on the weight basis of the tablet core; and
coating III: 6% of cellulose acetate and 3% of polyethylene glycol 4000, on the weight basis of the tablet core;
the tablet core consists of the drug-containing layer core and the boosting layer core; when the boosting layer core is absent, the tablet core is the drug-containing layer core.

9. A sacubitril valsartan sodium sustained-release preparation, comprising the sacubitril valsartan sustained-release composition according to any one of claims 1 to 8, wherein
preferably, the dosage form of the sacubitril valsartan sodium sustained-release preparation is a sustained-release tablet, a sustained-release pellet, or a sustained-release capsule.

10. Use of the sacubitril valsartan sustained-release composition according to any one of claims 1 to 8 or the sacubitril valsartan sustained-release preparation according to claim 9 for manufacturing a medicament for treating and/or preventing chronic heart failure with reduced ejection fraction.
